# EUROPEAN PATENT SPECIFICATION

(11) **EP 1 416 867 B1**
(45) Date of publication and mention of the grant of the patent: **09.12.2015**
(21) Application number: 02791531.3
(22) Date of filing: 29.04.2002
(51) Int. Cl.: A61B 17/56

(54) **SPINAL DISC ANNULUS RECONSTRUCTION**
BANDSCHEIBEN-REKONSTRUKTION
RECONSTRUCTION DE L'ANNULUS DU DISQUE INTERVERTEBRAL

(30) Priority: 31.07.2001 US 309105 P; 05.09.2001 US 947078; 15.02.2002 US 75615
(43) Date of publication of application: 12.05.2004
(73) Proprietor: KRT INVESTORS, INC., Trophy Club TX 46262 (US)
(72) Inventor: CAUTHEN, Joseph, C., III, Gainesville, FL 32607 (US); BURNS, Matthew, M., Orono, MN 55364 (US); WALES, Lawrence, W., Maplewood, MN 55119 (US); ADAMS, Daniel, O., Orono, MN 55356 (US); HOUFBURG, Rodney, L., Prior Lane, MN 55372 (US)
(74) Representative: Molnia, David
(86) International application number: PCT/US2002/013372
(87) International publication number: WO 2003/011155

## Description

### Cross-Reference to a Related Application

This application claims the benefit of U.S. Provisional Application No. 60/309,105, filed July 31, 2001. This application is also related to U.S. Patent Application 10/075,615, filed on February 15, 2002 and published as U.S 2002/0123 807.

### Field of the Invention

The invention generally relates to implantable medical devices for the closure, sealing and/or repair of an aperture in the intervertebral disc annulus. The term "aperture" refers to a hole in the annulus that is a result of a surgical incision into the intervertebral disc annulus, or the consequence of a naturally occurring tear (rent). The invention generally relates to surgical devices for intervertebral disc wall repair or reconstruction. The invention further relates to an annular repair device, or stent, for annular disc repair. These stents can be of natural or synthetic materials. The effects of said reconstruction are restoration of disc wall integrity and reduction of the failure rate (3-21 %) of a common surgical procedure (disc fragment removal or discectomy). This surgical procedure is performed about 390,000 times annually in the United States.

### Background of the Invention

The spinal column is formed from a number of bony vertebrae, which in their normal state are separated from each other by intervertebral discs. These discs are comprised of the annulus fibrosus, and the nucleus pulposus, both of which are soft tissue. The intervertebral disc acts in the spine as a crucial stabilizer, and as a mechanism for force distribution between adjacent vertebral bodies. Without the disc, collapse of the intervertebral space occurs in conjunction with abnormal joint mechanics and premature development of arthritic changes.

The normal intervertebral disc has an outer ligamentous ring called the annulus surrounding the nucleus pulposus. The annulus binds the adjacent vertebrae together and is constituted of collagen fibers that are attached to the vertebrae and cross each other so that half of the individual fibers will tighten as the vertebrae are rotated in either direction, thus resisting twisting or torsional motion. The nucleus pulposus is constituted of loose tissue, having about 85% water content, which moves about during bending from front to back and from side to side,

The aging process contributes to gradual changes in the intervertebral discs. The annulus loses much of its flexibility and resilience, becoming more dense and solid in composition. The aging annulus may also be marked by the appearance or propagation of cracks or fissures in the annular wall. Similarly, the nucleus desiccates, increasing viscosity and thus losing its fluidity. In combination, these features of the aged intervertebral discs result in less dynamic stress distribution because of the more viscous nucleus pulposus, and less ability to withstand localized stresses by the annulus fibrosus due to its desiccation, loss of flexibility and the presence of fissures. Fissures can also occur due to disease or other pathological conditions. Occasionally fissures may form rents through the annular wall. In these instances, the nucleus pulposus is urged outwardly from the subannular space through a rent, often into the spinal column. Extruded nucleus pulposus can, and often does, mechanically press on the spinal cord or spinal nerve rootlet. This painful condition is clinically referred to as a ruptured or herniated disc.

In the event of annulus rupture, the subannular nucleus pulposus migrates along the path of least resistance forcing the fissure to open further, allowing migration of the nucleus pulposus through the wall of the disc, with resultant nerve compression and leakage of chemicals of inflammation into the space around the adjacent nerve roots supplying the extremities, bladder, bowel and genitalia. The usual effect of nerve compression and inflammation is intolerable back or neck pain, radiating into the extremities, with accompanying numbness, weakness, and in late stages, paralysis and muscle atrophy, and/or bladder and bowel incontinence. Additionally, injury, disease or other degenerative disorders may cause one or more of the intervertebral discs to shrink, collapse, deteriorate or become displaced, herniated, or otherwise damaged and compromised.

The surgical standard of care for treatment of herniated, displaced or ruptured intervertebral discs is fragment removal and nerve decompression without a requirement to reconstruct the annular wall. While results are currently acceptable, they are not optimal. Various authors report 3.1- 21% recurrent disc herniation, representing a failure of the primary procedure and requiring re-operation for the same condition. An estimated 10% recurrence rate results in 39,000 re-operations in the United States each year.

An additional method of relieving the symptoms is thermal annuloplasty, involving the heating of sub-annular zones in the non-herniated painful disc, seeking pain relief, but making no claim of reconstruction of the ruptured, discontinuous annulus wall.

Some have also suggested that the repair of a damaged intervertebral disc might include the augmentation of the nucleus pulposus, and various efforts at nucleus pulposus replacement have been reported. The present invention is directed at the repair of the annulus, whether or not a nuclear augmentation is also warranted.

In addition, there has been experimentation in animals to assess various surgical incisions with and without the direct surgical repair of the annulus. These studies were performed on otherwise healthy animals and involved no removal or augmentation of nucleus pulposus. The authors of these experiments conclude that direct repair of the annulus does not influence the healing of the disc.

There is currently no known method of annulus reconstruction, either primarily or augmented with an annulus stent. WO 01/21246 A1 discloses a system for sealing a hole in a body.

### Brief Summary of the Invention

The present invention provides a device for occluding an aperture in the wall of an annulus fibrosus in cases of displaced, herniated, ruptured, or otherwise damaged intervertebral discs. In addition, a method is disclosed for intervertebral disc reconstruction for treating a disc having an aperture in the wall of the annulus fibrosis, wherein the aperture provides a path for the migration of nucleus pulposus from the subannular space, the method including the steps of providing an expandable patch having a first configuration dimensioned to pass through the aperture and a second expanded configuration having at least one dimension at least as large as the aperture and having at least one dimension larger than a corresponding dimension in said first configuration; inserting the patch through the aperture into the subannular space when the device is in the first collapsed configuration; and causing or allowing the patch to expand in the subannular space into the second expanded configuration to bridge the aperture, thereby occluding the aperture and preventing the migration of nucleus pulposus therethrough.

The objects and various advantages of the invention will be apparent in consideration of the description which follows. In general, the implantable medical device is placed, positioned, and affixed to the annulus to reduce re-extrusion of the nucleus through the aperture by: acting as a mechanical barrier; restoring the natural integrity of the wall of the annulus ; and, promoting the healing of the annulus through the reapproximation of disc wall tissue. Increased integrity and faster and/or more thorough healing of the aperture is intended to reduce future recurrence of herniation of the disc nucleus from the intervertebral disc, and the recurrence of resulting back pain. In addition, it is believed that the repair of the aperture could promote enhanced biomechanics and reduce the possibility of intervertebral disc height collapse and segmental instability, thus resulting in a decrease in the recurrence of back pain after a surgical procedure.

Moreover, the repair of the aperture with the reduction of the re-extrusion of the nucleus may also advantageously reduce adhesion formation surrounding the nerve roots. The nuclear material of the disc is toxic to the nerves and is believed to cause increased inflammation surrounding the nerves, which in turn can cause increased scar formation (adhesions or epidural fibrosis) upon healing. Adhesions created around the nerve roots can cause continued back pain. Any reduction in adhesion formation is believed to reduce future recurrence of pain.

One of the objects of the present inventions is to act as a mechanical barrier to the extrusion of the nucleus from the disc space, add mechanical integrity to the annulus and the tissue surrounding the aperture, and to promote faster and a more complete healing of the aperture.

Although much of the discussion is directed toward the repair of the intervertebral disc after a surgical procedure, such as discectomy (a surgical procedure performed to remove herniated fragments of the disc nucleus), it is contemplated that the device could be used in other procedures that involve incisions into the annulus of the intervertebral disc. An example of another procedure that could require a repair technique involves the replacement of the nucleus - nucleus replacement - with an implantable nucleus to replace the functioning of the natural nucleus when it is degenerated. The object of the invention in this case would be similar in that the repair would maintain the replacement nucleus within the disc space.

According to the invention, a sub-annular patch/stent can be employed to repair an intervertebral disc annulus. In its simplest form, the repair of the annulus involves the placement and fixation of a fascial autograft patch to the sub-annular space which can additionally employ two or more sutures, while re-approximating the tissues surrounding the aperture. The invention, through involvement of the sub-annular space and wall for the repair of the aperture has several advantages over the prior art, for example, sealing the aperture only on the outer surface or sealing the aperture only within the aperture. The first advantage of a repair that involves the sub-annular surface derives itself from the physical nature of a circular (or an elliptical) compressed chamber with a radius, like an intervertebral disc. Sealing the inside wall has the inherent advantage of being at a smaller radius of curvature versus the outer wall and thus, according to LaPlace's Law, the patch would be subjected to lower stresses at any given pressure, all else held equal.

Another advantage of utilizing the inner surface to accomplish sealing is that the natural pressure within the disc can enhance the sealing of the device against the inner wall of the disc space. Conversely, if the repair is performed on the outer surface of the annulus there is an inherent risk of leakage around the periphery of the device, with the constant exposure to the pressure of the disc.

Another advantage of the present invention over the prior art in utilizing the inner surface of the annulus is the reduction of the risk of having a portion of the device protruding from the exterior surface of the annulus. Device materials protruding from the exterior of the annulus pose a risk of damaging the nerve root and/or spinal canal which are in close proximity. Damage to these structures can result in continued pain, incontinence, bowel dysfunction and paralysis.

The present invention also incorporates the concept of pulling the tissues together that surround the aperture, the inner surface, and the outer surface of the annulus to help increase the integrity of the repair.

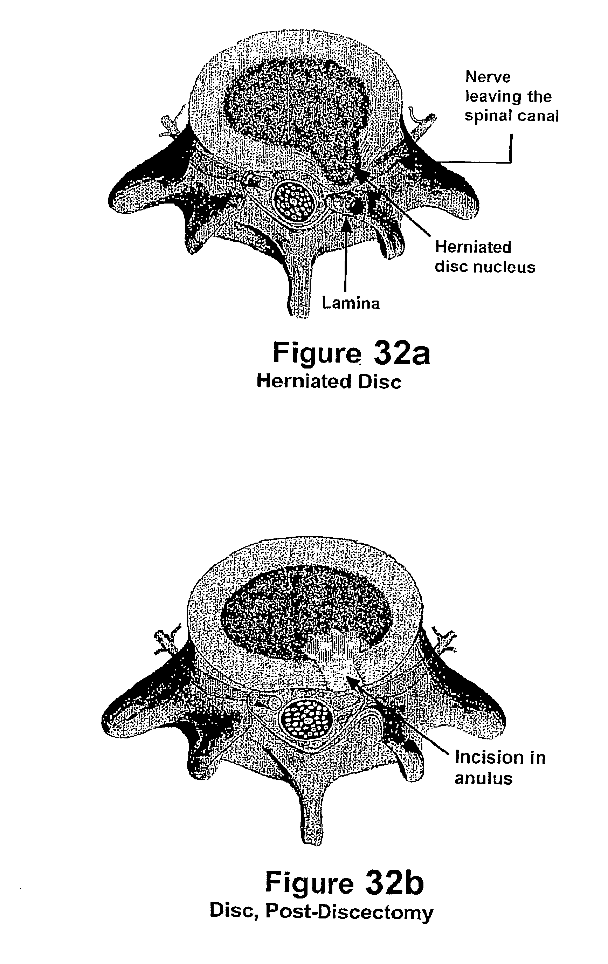

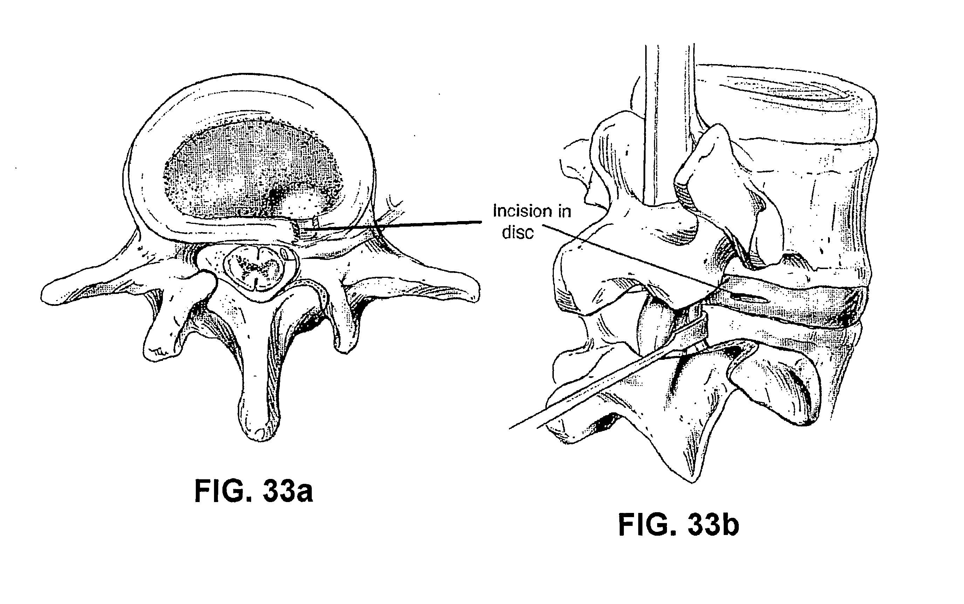

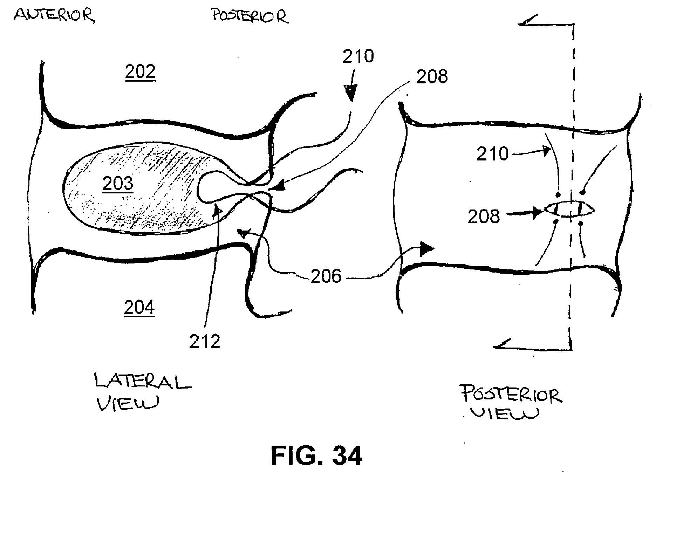

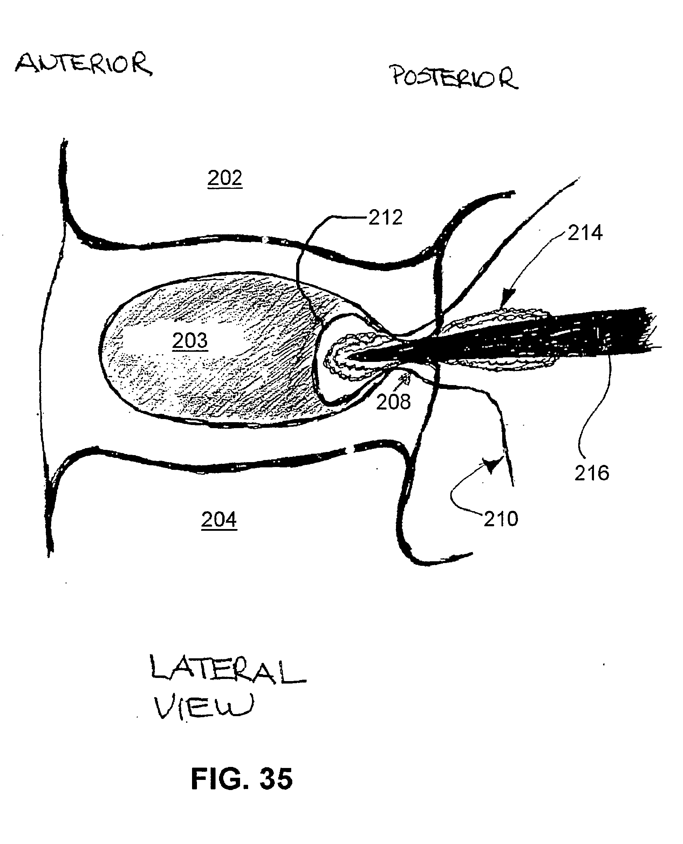

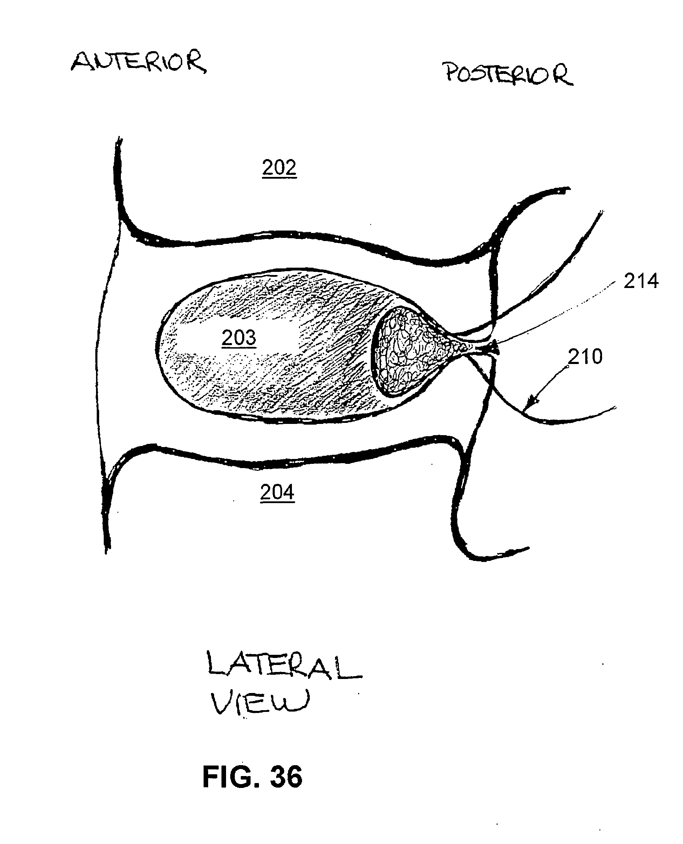

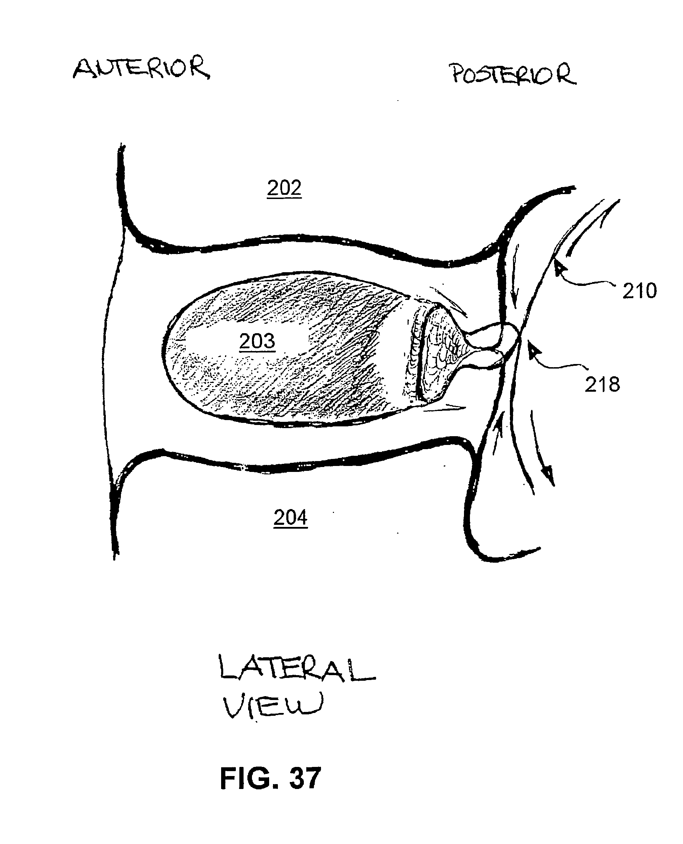

An example of the technique and placement of the device according to the invention is as follows:
1. An aperture is created measuring approximately, for example, 6 mm x 2 mm in the wall of the annulus after performing a discectomy procedure in which a portion of the nucleus is also removed from the disc space, as shown in FIGs. 32a, 32b, 33a and 33b.
2. Two or more sutures are passed through the upper and lower surfaces of the aperture and they are pushed within the intervertebral disc space to create a "sling" to receive the fascial autograft as shown for example in FIG. 34
3. A piece of para-spinal fascial tissue is removed from the patient measuring approximately, for example, 10 mm X 5 mm.
4. The autograft is folded and compressed to pass through the aperture in the annulus, as shown for example in FIG. 35
5. The autograft takes a second shape, within the annulus that is uncompressed and oriented to be in proximity of the subannular wall of the annulus, within the sling, as shown for example in FIG. 36. The autograft may be inserted entirely into the subannular space, or a portion may extend into the rent as depicted in FIG. 36.
6. The sutures are tightened, as shown for example in FIG. 37, thus tightening the sling surrounding the autograft, to bring the autograft in close proximity with the subannular wall, while providing tension to bring the patch at the subannular surface together with the outer surface of the annular wall, thus creating increased integrity of the annulus surrounding the aperture, as well as causing the autograft to take a second shape that is larger than the aperture. Furthermore, the tightening, and eventual tying of the sutures also promotes the re-approximation of the tissue at the outer surface of the annulus and within the aperture.
7. The sutures are tied and the ends of the sutures are cut.
8. A piece of autograft fat tissue may be placed over the discectomy site for the prevention of adhesion formation, a typical surgical technique.
9. Standard surgical techniques are utilized to close the access site of the surgical procedures.

Several devices according to the present invention can be used to practice the above illustrative inventive steps to accomplish the sealing and/or repair of the intervertebral disc. In each of the representative devices described herein, there is: an expandable patch/stent (note: patch, stent and device are used interchangeably) that has, in use, at least a portion of the device in proximity to the sub-annular space of the intervertebral disc annulus; a means to affix the patch to stay in proximity with the annulus; a means to draw the patch and the annular tissue together and fasten in tension; and a means to help reduce the relative motion of the surfaces of the aperture after fixation, and thus promote healing. According to one feature and object of the present invention, close approximation of tissue, while reducing the motion of the surfaces, provides the optimal environment for healing.

The concepts disclosed hereinbelow accomplish these objectives, as well as advantageously additionally incorporating design elements to reduce the number of steps (and time), and/or simplify the surgical technique, and/or reduce the risk of causing complications during the repair of the intervertebral disc annulus. In addition, it is an objective of the following devices to become incorporated by the surrounding tissues, or to act as a scaffold in the short-term (3 - 6 months) for tissue incorporation.

In an exemplary embodiment, one or more mild biodegradable surgical sutures can be placed at about equal distances along the sides of a pathologic aperture in the ruptured disc wall (annulus) or along the sides of a surgical incision in the annular wall, which may be weakened or thinned.

Sutures are then tied in such fashion as to draw together the sides of the aperture, effecting reapproximation or closure of the opening, to enhance natural healing and subsequent reconstruction by natural tissue (fibroblasts) crossing the now surgically narrowed gap in the disc annulus.

A 25-30% reduction in the rate of recurrence of disc nucleus herniation through this aperture has been achieved using this method.

In another exemplary embodiment, the method can be augmented by creating a subannular barrier in and across the aperture by placement of a patch of human muscle fascia (muscle connective tissue) or any other autograft, allograft, or xenograft acting as a bridge or a scaffold, providing a platform for traverse of fibroblasts or other normal cells of repair existing in and around the various layers of the disc annulus, prior to closure of the aperture.

A 30-50% reduction in the rate of recurrence of disc herniation has been achieved using the aforementioned fascial augmentation with this embodiment.

Having demonstrated that human muscle fascia is adaptable for annular reconstruction, other biocompatible membranes can be employed as a bridge, stent, patch or barrier to subsequent migration of the disc nucleus through the aperture. Such biocompatible materials may be, for example, medical grade biocompatible fabrics, biodegradable polymeric sheets, or form fitting or non-form fitting fillers for the cavity created by removal of a portion of the disc nucleus pulposus in the course of the disc fragment removal or discectomy. The prosthetic material can be placed in and around the intervertebral space, created by removal of the degenerated disc fragments.

Additional objects and advantages of the invention will be set forth in part in the description which follows, and in part will be obvious from the description, or may be learned by practice of the invention. The objects and advantages of the invention will be realized and attained by means of the elements and combinations particularly pointed out in the appended claims.

It is to be understood that both the foregoing general description and the following detailed description are exemplary and explanatory only and are not restrictive of the invention, as claimed.

### Brief Description of the Drawings

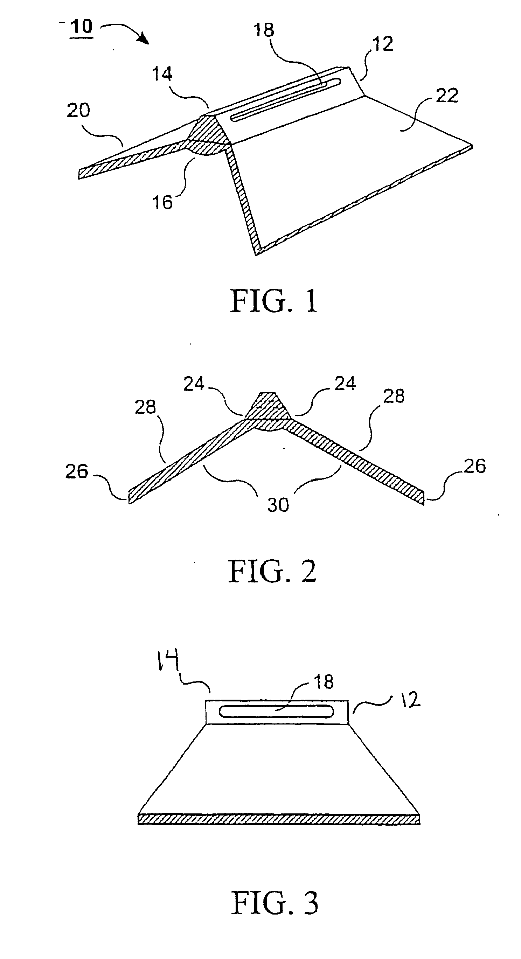

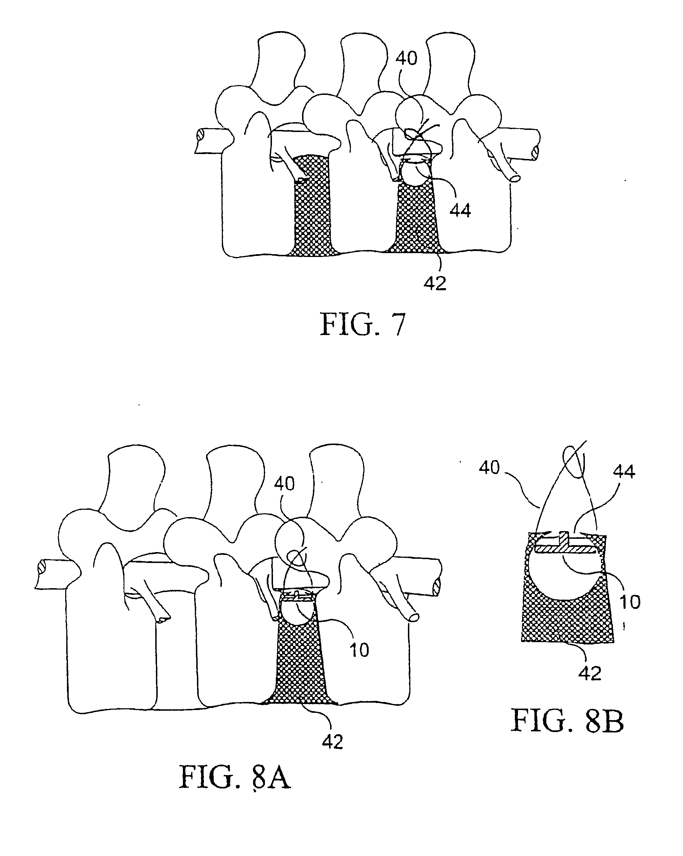

The accompanying drawings, which are incorporated in and constitute a part of this specification, are meant to illustrate illustrative embodiments of the invention and, together with the description, serve to explain the principles of the invention. Figures 1 - 6, 10 and 13 - 59 show embodiments according to the invention and procedures in which the inventive embodiments may be used. Figures 7, 8, 9, 11 and 12 show exemplary embodiments.
FIG. 1 shows a perspective view of an illustrative embodiment of an annulus stent.
FIG. 2 shows a front view of the annulus stent of FIG. 1.
**FIG. 3** shows a side view of the annulus stent of FIG. 1.
**FIGs. 4A-4C** show a front view of alternative illustrative embodiments of an annulus stent.
**FIGs. 5A-5B** show the alternative embodiment of a further illustrative embodiment of an annulus stent.
**FIGs. 6A-6B** show the alternative embodiment of a further illustrative embodiment of an annulus stent.
**FIG. 7** shows a primary closure of an opening in the disc annulus.
**FIGs. 8A-8B** show a primary closure with a stent.
**FIG. 9** shows a method of suturing an annulus stent into the disc annulus utilizing fixation points on vertebral bodies.
**FIGs. 10A-10B** show a further illustrative embodiment of an annulus stent with flexible bladder being expanded into the disc annulus.
**FIGs. 11A-11D** show an annulus stent being inserted into and expanded within the disc annulus.
**FIGs. 12A- 12B** show an annulus stent with a flexible bladder being expanded.
**FIG. 13** shows a perspective view of a further illustrative embodiment of an annulus stent.
**FIG. 14** shows a first collapsed view of the annulus stent of FIG. 13.
**FIG. 15** shows a second collapsed view of the annulus stent of FIG. 13.
**FIGs. 16A-16C** show the annulus stent of FIG. 13 being inserted into the disc annulus.
**FIGs. 17A-17C** show a method of inserting the annulus stent of FIG. 13 into the disc annulus.
**FIGs. 18A-18B** show a further illustrative embodiment of an annulus stent with a flexible bladder.
**FIGs. 19A-19B** show another illustrative embodiment of an annulus stent with a flexible bladder.
**FIG. 20** shows an expanded annulus stent with barbs on the radial extension.
**FIG. 21** shows a still further illustrative embodiment of an annulus stent with a compressible core.
**FIG. 22** shows a still further illustrative embodiment of an introduction device for an annulus stent.
**FIG. 23** shows a modification of the device depicted in FIG. 22.
**FIG. 24** shows an exemplary introduction tool for use with the devices of FIGs. 22 and 23 with a stent deflected proximally.
**FIG. 25** shows an exemplary introduction tool for use with the devices of FIGs. 22 and 23 with a stent deflected distally.
**FIG. 26** shows an exemplary introduction tool for use with the devices of FIGs. 22 and 23 with a stent deflected partially distally and partially proximally.
**FIG. 27** shows a still further illustrative embodiment of a stent device having a grasping feature and fixation devices in the form of barbs.
**FIG. 28** shows the illustrative embodiment in FIG. 27 deployed subannularly.
**FIG. 29** shows a still further illustrative embodiment of an annulus stent employing a secondary barbed fixation device.
**FIG. 30** shows a still further illustrative embodiment of an annulus stent employing another example of a secondary barbed fixation device.
**FIG. 31** shows the frame of a still further illustrative embodiment of an annulus stent having a metal substrate being machined from flat stock.
**FIG. 32a** shows a herniated disc in perspective view, and **FIG. 32b** shows the same disc after discectomy.
**FIG. 33a** shows a top view of the disc post-discectomy, and **FIG. 33b** shows a posteriolateral view of the disk showing an incision.
**FIG. 34** shows schematically the creation of a subannular sling using sutures.
**FIG. 35** schematically shows the introduction of a compressed autograft stent/patch into the subannular space.
**FIG. 36** schematically shows the autograft of FIG. 35 in an expanded shape within the annulus.
**FIG. 37** schematically shows the tightening of the sutures to reappoximate the annulus aperture and draw the stent/patch of FIG. 35 toward the annular wall.
**FIG. 38** shows an exemplary collar for use in repairing a disc annulus.
**FIG. 39** schematically depicts the collar of FIG. 38 in use for disc annulus repair.
**FIG. 40** shows a still further exemplary embodiment of the present invention using a bag to contain the patch/stent.
**FIG. 41a****-e** show still further illustrative embodiments of the present invention having frames.
**FIG. 42** shows an illustrative method for placing a barbed expandable patch in the subannular disc space.
**FIG. 43** shows the patch of FIG. 42 being fixed to the inside wall of the annulus fibrosus.
**FIGs. 44a****-g** show a still further illustrative embodiment of an introduced and expanded annulus stent/patch being fixated and the aperture reapproximated.
**FIGs. 45a****-c** schematically depict a still further embodiment of the present invention where an expandable stent/patch is tethered in situ using a cinch line.
**FIGs. 46a****-c** schematically depict the cinch line of FIG. 45 being fixated through use of a surgical staple device.
**FIGs. 47a****-b** show an illustrative embodiment of a suturing arrangement for securing a patch/stent in the annulus.
**FIG. 48a****-b** depict a still further illustrative embodiment where fixation sutures are placed into the vertebral body or the Sharpey fibers.
**FIGs. 49a****-c** schematically depict a still further embodiment of the present invention where an expandable stent/patch is tethered in situ using a cinch line.
**FIGs. 50a****-c** schematically depict the cinch line of FIG. 49 being fixated through use of a barbed surgical staple device that penetrates the patch/stent.
**FIG. 51** depicts an exemplary use of filler tissue within the aperture during placement of a patch/stent tethered by a cinch line.
**FIGs. 52a****-e** shows exemplary embodiments of various additional patch/stent fixation techniques.
**FIG. 53** shows a still further illustrative embodiment of a stent/patch having a frame.
**FIG. 54a****-f** shows a still further illustrative embodiment of an annular stent/patch having a self-contained fixation tightening feature.
**FIG. 55** shows a still further exemplary embodiment of the present invention having external fixation anchors.
**FIG. 56a****-c** shows a still further exemplary embodiment of the present invention having external fixation anchors.
**FIG. 57a****-c** shows a still further exemplary embodiment of the present invention having external fixation anchors.
**FIG. 58** shows a still further exemplary embodiment of the present invention having external fixation anchors.
**FIG. 59** shows a still further exemplary embodiment of the present invention having a springing arrangement.

### Detailed Description of the Invention

Reference will now be made in detail to an illustrative embodiment of the invention, which appears in the accompanying drawings. Wherever possible, the same reference numbers will be used throughout the drawings to refer to the same or like parts.

In one embodiment of the present invention, as shown in FIG. 7, a damaged annulus **42** is repaired by use of surgical sutures **40.** One or more surgical sutures **40** are placed at about equal distances along the sides of a pathologic aperture **44** in the annulus **42.** Reapproximation or closure of the aperture **44** is accomplished by tying the sutures **40** so that the sides of the aperture **44** are drawn together. The reapproximation or closure of the aperture **44** enhances the natural healing and subsequent reconstruction by the natural tissue (e.g., fibroblasts) crossing the now surgically narrowed gap in the annulus **42.** Preferably, the surgical sutures **40** are biodegradable, but permanent non- biodegradable may be utilized.

Additionally, to repair a weakened or thinned wall of a disc annulus **42,** a surgical incision can be made along the weakened or thinned region of the annulus **42** and one or more surgical sutures **40** can be placed at about equal distances laterally from the incision. Reapproximation or closure of the incision is accomplished by tying the sutures **40** so that the sides of the incision are drawn together. The reapproximation or closure of the incision enhances the natural healing and subsequent reconstruction by the natural tissue crossing the now surgically narrowed gap in the annulus **42.** Preferably, the surgical sutures **40** are biodegradable, but permanent non-biodegradable materials may be utilized.

In an alternative embodiment, the method can be augmented by the placement of a patch of human muscle fascia or any other autograft, allograft or xenograft in and across the aperture **44.** The patch acts as a bridge in and across the aperture **44,** providing a platform for traverse of fibroblasts or other normal cells of repair existing in and around the various layers of the disc annulus **42,** prior to closure of the aperture **44.**

In a further embodiment, as shown in FIGs. 8A-B a biocompatible membrane can be employed as an annulus stent **10,** being placed in and across the aperture **44.** The annulus stent **10** acts as a bridge in and across the aperture **44,** providing a platform for a traverse of fibroblasts or other normal cells of repair existing in and around the various layers of the disc annulus **42,** prior to closure of the aperture **44.** In some embodiments the device, stent or patch can act as a scaffold to assist in tissue growth that healingly scars the annulus.

In an illustrative embodiment, as shown in FIGs. 1-3, the annulus stent **10** comprises a centralized vertical extension **12,** with an upper section **14** and a lower section **16.** The centralized vertical extension **12** can be trapezoid in shape through the width and may be from about 8mm - 12mm in length.

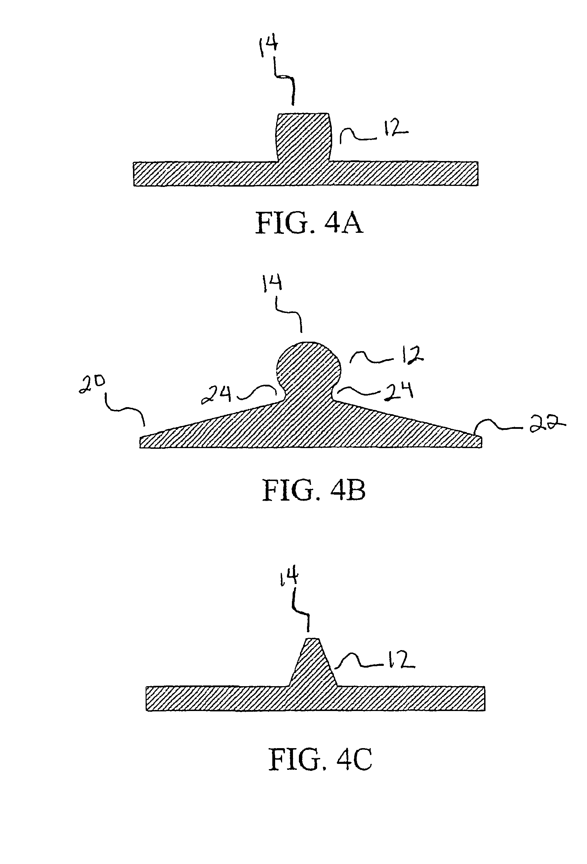

Additionally, the upper section **14** of the centralized vertical extension **12** may be any number of different shapes, as shown in FIGs. 4A through 4C, with the sides of the upper section **14** being curved or with the upper section **14** being circular in shape. Furthermore, the annulus stent **10** may contain a recess between the upper section **14** and the lower section **16,** enabling the annulus stent **10** to form a compatible fit with the edges of the aperture **44.**

The upper section **14** of the centralized vertical extension **12** can comprise a slot **18,** where the slot **18** forms an orifice through the upper section **14.** The slot **18** is positioned within the upper section **14** such that it traverses the upper section's **14** longitudinal axis. The slot **18** is of such a size and shape that sutures, tension bands, staples or any other type of fixation device known in the art may be passed through, to affix the annulus stent **10** to the disc annulus **42.**

In an alternative embodiment, the upper section **14** of the centralized vertical extension **12** may be perforated. The perforated upper section **14** contains a plurality of holes that traverse the longitudinal axis of upper section **14.** The perforations are of such a size and shape that sutures, tension bands, staples or any other type of fixation device known in the art may be passed through, to affix the annulus stent 10 to the disc annulus **42.**

The lower section **16** of the centralized vertical extension **12** can comprise a pair of lateral extensions, a left lateral extension **20** and a right lateral extension **22.** The lateral extensions **20** and **22** comprise an inside edge **24,** an outside edge **26,** an upper surface **28,** and a lower surface **30.** The lateral extensions **20** and **22** can have an essentially constant thickness throughout. The inside edge **24** is attached to and is about the same length as the lower section **16.** The outside edge **26** can be about 8mm-16mm in length. The inside edge **24** and the lower section **16** meet to form a horizontal plane, essentially perpendicular to the centralized vertical extension **12.** The upper surface **28** of the lateral extensions **20** and **22** can form an angle from about 0°-60° below the horizontal plane. The width of the annulus stent **10** may be from about 3mm-8mm.

Additionally, the upper surface **28** of the lateral extensions **20** and **22** may be barbed for fixation to the inside surface of the disc annulus 42 and to resist expulsion through the aperture **44.**

In an alternative embodiment, as shown in FIG. 4B, the lateral extensions **20** and **22** have a greater thickness at the inside edge **24** than at the outside edge **26.**

In an illustrative embodiment, the annulus stent **10** is a solid unit, formed from one or more of the flexible resilient biocompatible or bioresorbable materials well know in the art. The selection of appropriate stent materials may be partially predicated on specific stent construction and the relative properties of the material such that, after fixed placement of the stent, the repair may act to enhance the healing process at the aperture by relatively stabilizing the tissue and reducing movement of the tissue surrounding the aperture.

For example, the annulus stent **10** may be made from:

A porous matrix or mesh of biocompatible and bioresorbable fibers acting as a scaffold to regenerate disc tissue and replace annulus fibrosus as disclosed in, for example, U, S. Patent Nos. 5,108,438 (Stone) and 5,258,043 (Stone), a strong network of inert fibers intermingled with a bioresorbable (or bioabsorable) material which attracts tissue ingrowth as disclosed in, for example, U.S. Patent No, 4,904,260 (Ray et al.).

a biodegradable substrate as disclosed in, for example, U.S. Patent No. 5,964,807 (Gan at al.); or

an expandable polytetrafluoroethylene (ePTFE), as used for conventional vascular grafts, such as those sold by W.L. Gore and Associates, Inc. under the trademarks GORE-TEX and PRECLUDE, or by Impra, Inc. under the trademark IMPRA.

Furthermore, the annulus, stent **10,** may contain hygroscopic material for a controlled limited expansion of the annulus stent **10** to fill the evacuated disc space cavity.

Additionally, the annulus stent **10** may comprise materials to facilitate regeneration of disc tissue, such as bioactive silica-based materials that assist in regeneration of disc tissue as disclosed in U.S. Patent No. 5,849,331 (Ducheyne, et al.), or other tissue growth factors well known in the art.

Many of the materials disclosed and described above represent embodiments where the device actively promotes the healing process. It is also possible that the selection of alternative materials or treatments may modulate the role in the healing process, and thus promote or prevent healing as may be required. It is also contemplated that these modulating factors could be applied to material substrates of the device as a coating, or similar covering, to evoke a different tissue response than the substrate without the coating.

In further embodiments, as shown in FIGs. 5AB-6AB, the left and right lateral extensions **20** and **22** join to form a solid pyramid or cone. Additionally, the left and right lateral extensions **20** and **22** may form a solid trapezoid, wedge, or bullet shape. The solid formation may be a solid biocompatible or bioresorbable flexible material, allowing the lateral extensions **20** and **22** to be compressed for insertion into aperture **44,** then to expand conforming to the shape of the annulus' **42** inner wall.

Alternatively, a compressible core may be attached to the lower surface **30** of the lateral extensions **20** and **22,** forming a pyramid, cone, trapezoid, wedge, or bullet shape. The compressible core may be made from one of the biocompatible or bioresorbable resilient foams well known in the art. The core can also comprise a fluid-expandable membrane, e.g., a balloon. The compressible core allows the lateral extensions **20** and **22** to be compressed for insertion into aperture **44,** then to expand conforming to the shape of the annulus' **42** inner wall and to the cavity created by pathologic extrusion or surgical removal of the disc fragment.

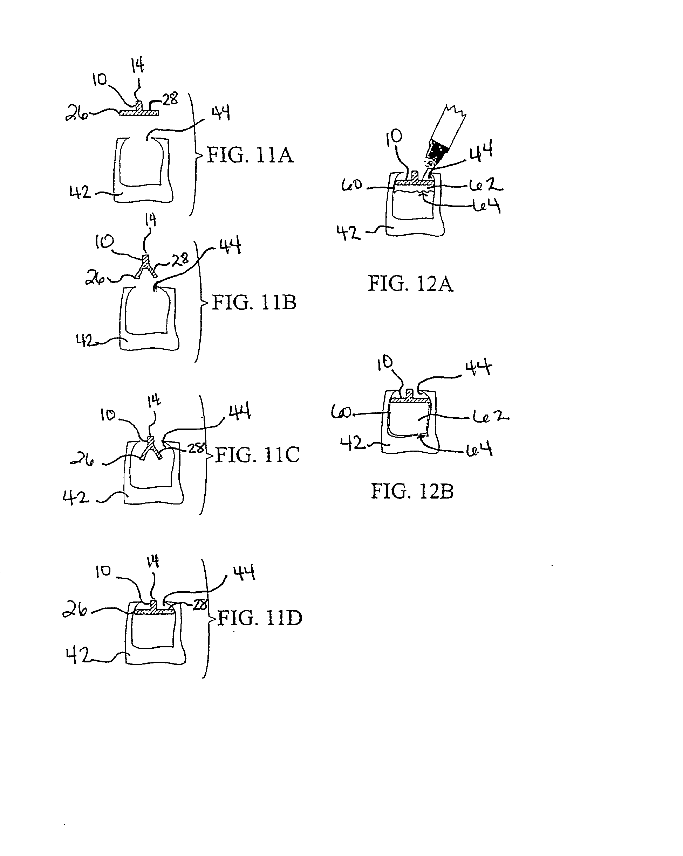

In an illustrative method of use, as shown in FIGs. 11A-D, the lateral extensions **20** and **22** are compressed together for insertion into the aperture **44** of the disc annulus **42.** The annulus stent **10** is then inserted into the aperture **44,** where the lateral extensions **20, 22** expand. In an expanded configuration, the upper surface **28** can substantially conform to the contour of the inside surface of the disc annulus 42. The upper section **14** is positioned within the aperture **44** so that the annulus stent **10** may be secured to the disc annulus **42,** using means well known in the art.

In an alternative method, where the length of the aperture **44** is less than the length of the outside edge **26** of the annulus stent **10,** the annulus stent **10** can be inserted laterally into the aperture **44.** The lateral extensions **20** and **22** are compressed, and the annulus stent **10** can then be laterally inserted into the aperture **44.** The annulus stent **10** can then be rotated inside the disc annulus **42,** such that the upper section **14** can be held back through the aperture **44.** The lateral extensions **20** and **22** are then allowed to expand, with the upper surface **28** contouring to the inside surface of the disc annulus **42.** The upper section **14** can be positioned within, or proximate to, the aperture **44** in the subannular space such that the annulus stent **10** may be secured to the disc annulus, using means well known in the art.

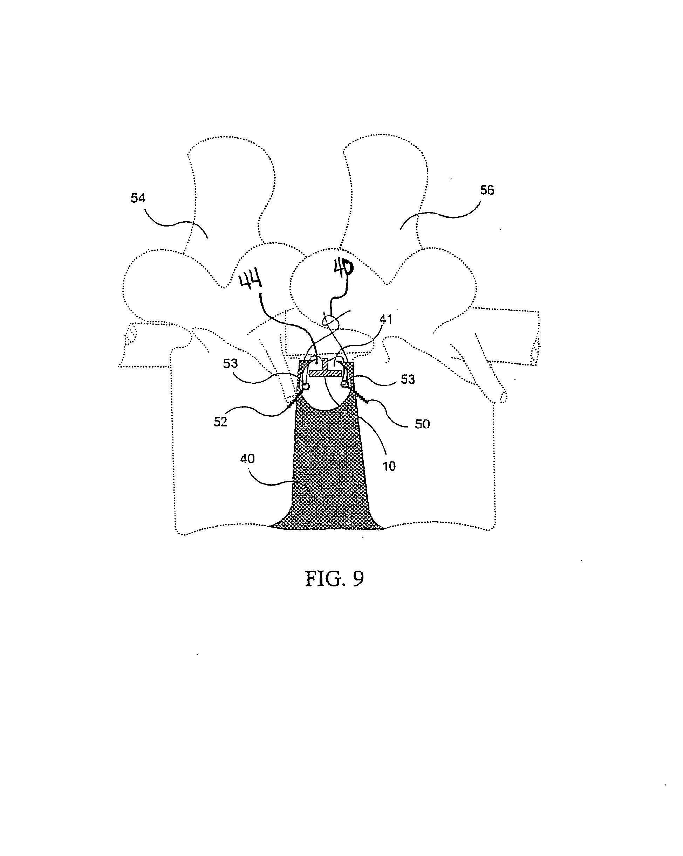

In an alternative method of securing the annulus stent **10** in the aperture **44,** as shown in FIG. 9, a first surgical screw **50** and second surgical screw **52,** with eyeholes **53** located at the top of the screws **50** and **52,** are inserted into the vertebral bodies, illustratively depicted as adjacent vertebrae **54** and **56.** After insertion of the annulus stent **10** into the aperture **44,** a suture **40** is passed down though the disc annulus **42,** adjacent to the aperture **44,** through the eye hole **53** on the first screw **50** then back up through the disc annulus **42** and through the orifice **18** on the annulus stent **10.** This is repeated for the second screw **52,** after which the suture **40** is secured. One or more surgical sutures **40** are placed at about equal distances along the sides of the aperture **44** in the disc annulus **42.** Reapproximation or closure of the aperture **44** is accomplished by tying the sutures **40** in such a fashion that the sides of the aperture **44** are drawn together. The reapproximation or closure of the aperture **44** enhances the natural healing and subsequent reconstruction by the natural tissue crossing the now surgically narrowed gap in the annulus **42.** Preferably, the surgical sutures **40** are biodegradable but permanent non-biodegradable forms may be utilized. This method should decrease the strain on the disc annulus **42** adjacent to the aperture **44,** precluding the tearing of the sutures through the disc annulus **42.**

It is anticipated that fibroblasts will engage the fibers of the polymer or fabric of the intervertebral disc stent **10,** forming a strong wall duplicating the currently existing condition of healing seen in the normal reparative process.

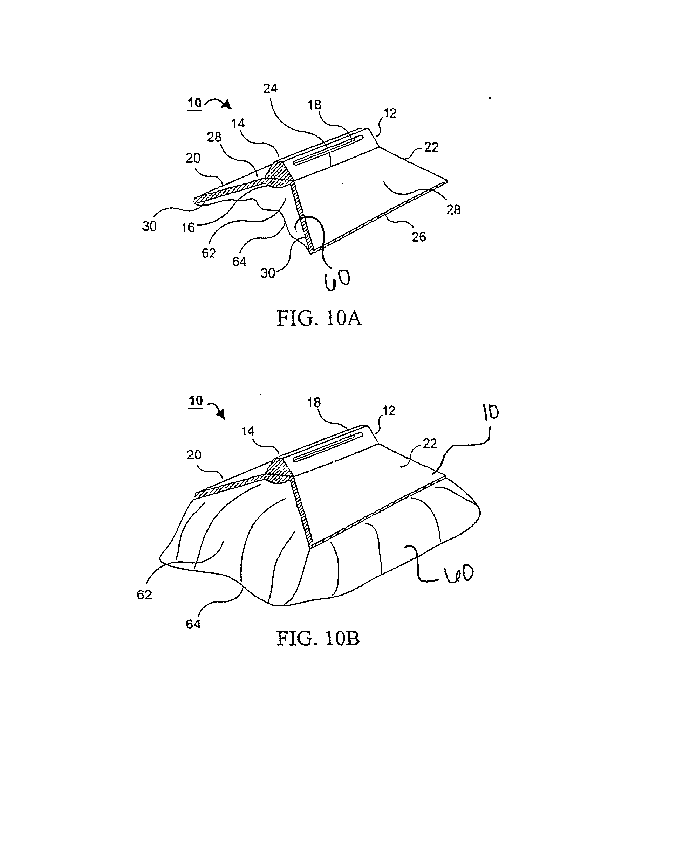

In an additional embodiment, as shown in FIGs. 10A-B, a flexible bladder **60** is attached to the lower surface **30** of the annulus stent **10.** The flexible bladder **60** comprises an internal cavity **62** surrounded by a membrane **64,** where the membrane **64** is made from a thin flexible biocompatible material. The flexible bladder **60** is attached to the lower surface **30** of the annulus stent **10** in an unexpanded condition. The flexible bladder **60** is expanded by injecting a biocompatible fluid or expansive foam, as known in the art, into the internal cavity **62.** The exact size of the flexible bladder **60** can be varied for different individuals. The typical size of an adult nucleus is about 2 cm in the semi-minor axis, 4 cm in the semi-major axis, and 1.2 cm in thickness.

In an alternative embodiment, the membrane **64** is made of a semi-permeable biocompatible material. The mechanical properties of the injectate material may influence the performance of the repair and it is contemplated that materials which are "softer" or more compliant as well as materials that are less soft and less compliant than healthy nucleus are contemplated within the scope of certain embodiments of the invention. It must be understood that in certain embodiments the volume added to the subannular space may be less than equal to or larger than the nucleus volume removed. The volume of the implant may vary over time as well in certain embodiments.

In an illustrative embodiment, a hydrogel is injected into the internal cavity **62** of the flexible bladder **60.** A hydrogel is a substance formed when an organic polymer (natural or synthetic) is cross-linked via, covalent, ionic, or hydrogen bonds to create a three-dimensional open-lattice structure, which entraps water molecules to form a gel. The hydrogel may be used in either the hydrated or dehydrated form.

In a method of use, where the annulus stent **10** has been inserted into the aperture **44,** as has been previously described and shown in FIGs. 12 A-B, an injection instrument, as known in the art, such as a syringe, is used to inject the biocompatible fluid or expansive foam into the internal cavity **62** of the flexible bladder **60.** The biocompatible fluid or expansive foam is injected through the annulus stent **10** into the internal cavity **62** of the flexible bladder **60.** Sufficient material is injected into the internal cavity **62** to expand the flexible bladder **60** to fill the void in the intervertebral disc cavity. The use of the flexible bladder **60** is particularly useful when it is required to remove all or part of the intervertebral disc nucleus.

The surgical repair of an intervertebral disc may require the removal of the entire disc nucleus, being replaced with an implant, or the removal of a portion of the disc nucleus thereby leaving a void in the intervertebral disc cavity. The flexible bladder **60** allows for the removal of only the damaged section of the disc nucleus, with the expanded flexible bladder **60** filling the resultant void in the intervertebral disc cavity. A major advantage of the annulus stent **10** with the flexible bladder **60** is that the incision area in the annulus **42** can be reduced in size, as there is no need for the insertion of an implant into the intervertebral disc cavity.

In an alternative method of use, a dehydrated hydrogel is injected into the internal cavity **62** of the flexible bladder **60.** Fluid, from the disc nucleus, passes through the semipermeable membrane **64** hydrating the dehydrated hydrogel. As the hydrogel absorbs the fluid the flexible bladder **60** expands, filling the void in the intervertebral disc cavity.

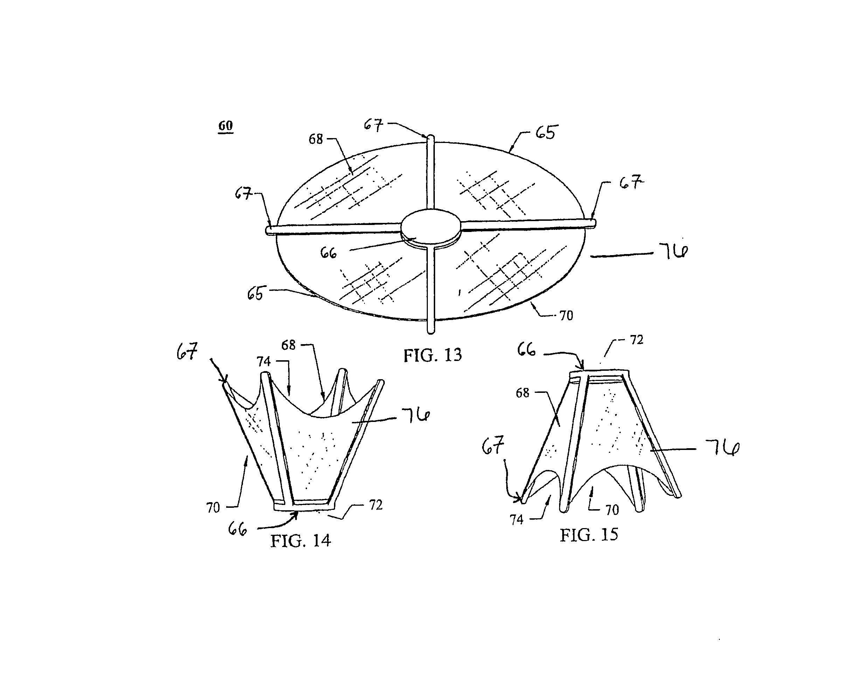

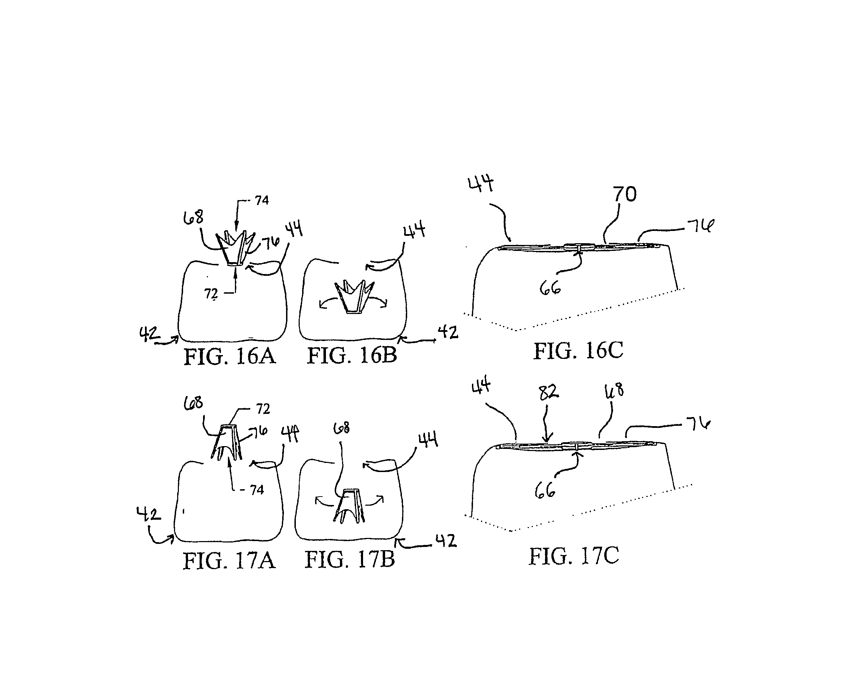

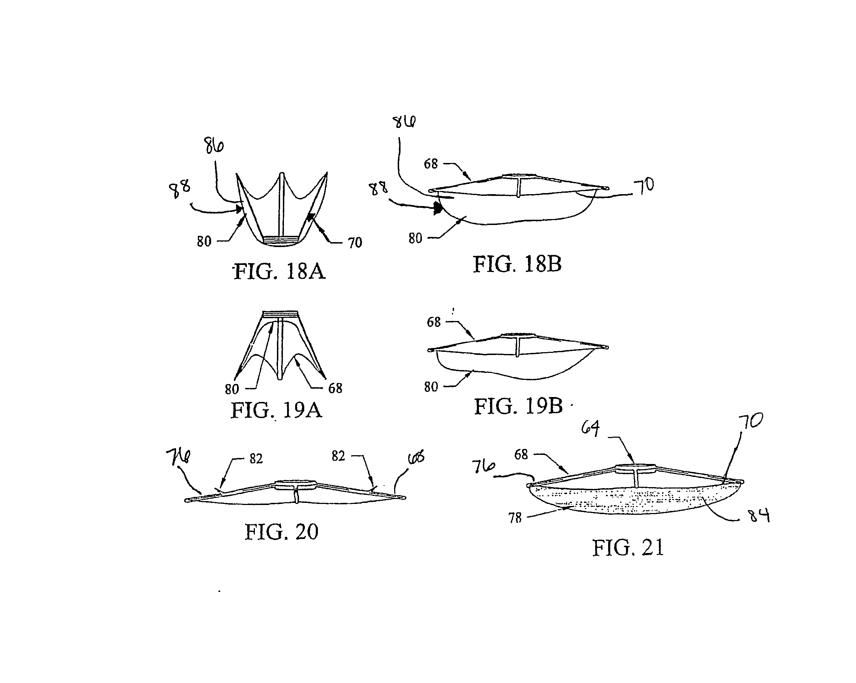

In an alternative embodiment, as shown in FIG. 13, the annulus stent **10** is substantially umbrella shaped, having a central hub **66** with radially extending struts **67.** Each of the struts **67** is joined to the adjacent struts **67** by a webbing material **65,** forming a radial extension **76** about the central hub **66.** The radial extension **76** has an upper surface **68** and a lower surface **70,** where the upper surface **68** contours to the shape of the disc annulus' **42** inner wall when inserted as shown in FIG. 17A-C, and where the lower surface **70** contours to the shape of the disc annulus' **42** inner wall when inserted as shown in FIG. 16A-C. The radial extension **76** may be substantially circular, elliptical, or rectangular in plan shape. Additionally, as shown in FIG. 20, the upper surface **68** of the radial extension **76** may be barbed **82** for fixation to the disc annulus' **42** inner wall and to resist expulsion through the aperture **42.**

As shown in FIGs. 14 and 15, the struts **67** are formed from flexible material, allowing the radial extension **76** to be collapsed for insertion into aperture **44,** then the expand conforming to the shape of the inner wall of disc annulus **42.** In the collapsed position, the annulus stent **10** is substantially frustoconical or shuttlecock shaped, and having a first end **72,** comprising the central hub **66,** and a second end **74.**

In an alternative embodiment, the radial extension **76** has a greater thickness at the central hub **66** edge than at the outside edge.

In an embodiment, the annulus stent **10** is a solid unit, formed from one or more of the flexible resilient biocompatible or bioresorbable materials well known in the art.

Additionally, the annulus stent **10** may comprise materials to facilitate regeneration of disc tissue, such as bioactive silica based materials that assist in regeneration of disc tissue as disclosed in U.S. Patent No. 5,849,331 (Ducheyne, et al.), or other tissue growth factors well known in the art.

Alternatively, as shown in FIG. 21, a compressible core **84** may be attached to the lower surface **70** of the radial extension **76.** The compressible core **84** may be made from one of the biocompatible or bioresorbable resilient foams well known in the art. The compressible core **84** allows the radial extension **76** to be compressed for insertion into aperture **44** then to expand conforming to the shape of the disc annulus' **42** inner wall and to the cavity created by pathologic extrusion or surgical removal of the disc fragment.

In an additional embodiment, as shown in FIG. 18A and 18B, a flexible bladder **80** is attached to the lower surface **70** of the annulus stent **10.** The flexible bladder **80** comprises an internal cavity **86** surrounded by a membrane **88,** where the membrane **88** is made from a thin flexible biocompatible material. The flexible bladder **86** is attached to the lower surface **70** of the annulus stent **10** in an unexpanded condition. The flexible bladder **80** is expanded by injecting a biocompatible fluid or expansive foam, as known in the art, into the internal cavity **86.** The exact size of the flexible bladder **80** can be varied for different individuals. The typical size of an adult nucleus is 2 cm in the semi-minor axis, 4 cm in the semi-major axis and 1.2 cm in thickness.

In an alternative embodiment, the membrane **88** is made of a semi-permeable biocompatible material.

In a method of use, as shown in FIGs. 16A-16C, the radial extension **76** is collapsed together, for insertion into the aperture **44** of the disc annulus **42.** The radial extension **76** is folded such the upper surface **68** forms the outer surface of the cylinder. The annulus stent **10** is then inserted into the aperture **44,** inserting the leading end **72** though the aperture **44** until the entire annulus stent **10** is within the disc annulus **42.** The radial extension **76** is released, expanding within the disc **44.** The lower surface **70** of the annulus stent **10** contours to the inner wall of disc annulus **42.** The central hub **66** is positioned within the aperture **44** so that the annulus stent **10** may be secured to the disc annulus **42** using means well known in the art.

It is anticipated that fibroblasts will engage the fibers of the polymer of fabric of the annulus stent **10,** forming a strong wall duplicating the currently existing condition of healing seen in the normal reparative process.

In an alternative method of use, as shown in FIGs. 17A-17C, the radial extension **76** is collapsed together for insertion into the aperture **44** of the disc annulus **42.** The radial extension **76** is folded such that the upper surface **68** forms the outer surface of the stent, for example in a frustoconical configuration as illustrated. The annulus stent **10** is then inserted into the aperture **44,** inserting the tail end **74** through the aperture **44** until the entire annulus stent **10** is in the disc. The radial extension **76** is released, expanding within the disc. The upper surface **68** of the annulus stent **10** contours to the disc annulus' **42** inner wall. The central hub **66** is positioned within the aperture **44** so that the annulus stent **10** may be secured to the disc annulus **42,** using means well known in the art.

In one illustrative embodiment, the barbs **82** on the upper surface **68** of one or more strut **67** or other feature of the radial extension **76,** engage the disc annulus' **42** inner wall, holding the annulus stent **10** in position.

In a method of use, as shown in FIGs. 12A-12B, where the annulus stent **10** has been inserted into the aperture **44,** as has been previously described. Similarly, for the stent shown in FIGs. 18 through 21, an injection instrument, as known in the art, such as a syringe, can be used to inject the biocompatible fluid or expansive foam into the internal cavity **86** of the flexible bladder **80.** The biocompatible fluid or expansive foam is injected through the annulus stent **10** into the internal cavity **86** of the flexible bladder **80.** Sufficient material is injected into the internal cavity **86** to expand the flexible bladder **80** to fill the void in the intervertebral disc cavity. The material can be curable (*i.e.,* glue). The use of the flexible bladder **80** is particularly useful when it is required to remove all or part of the intervertebral disc nucleus.

It should be noted that in any of the "bag" embodiments described herein one wall or barrier can be made stiffer and less resilient than others. This relatively stiff wall member can then be placed proximate the annulus wall and can advantageously promote, in addition to its reparative properties, bag containment within the annulus.

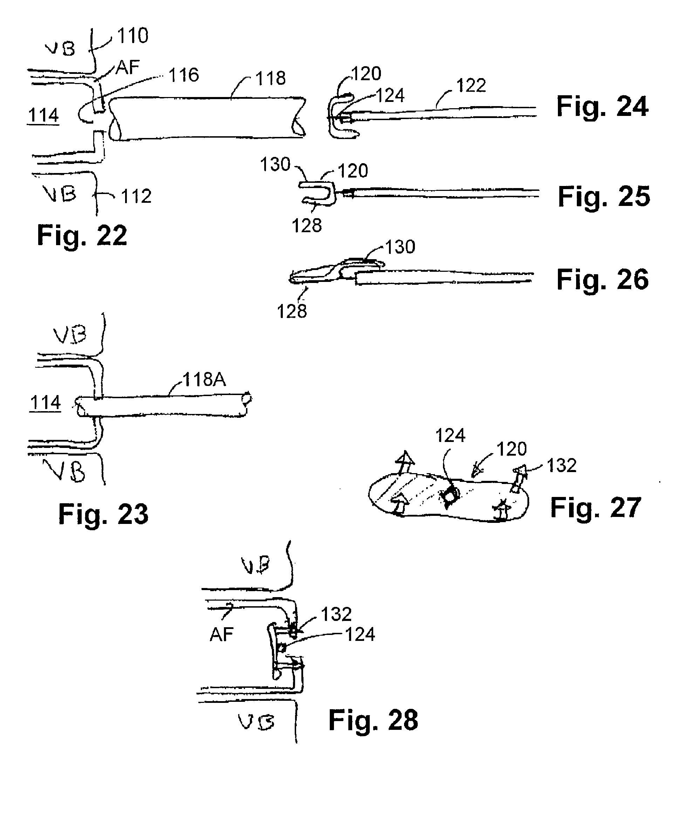

FIG. 22 shows a further aspect of the present invention. According to a further illustrative embodiment, a simplified schematic cross section of a vertebral pair is depicted including an upper vertebral body **110,** a lower vertebral body **112** and an intervertebral disc **114.** An aperture or rent **116** in the annulus fibrosus **(AF)** is approached by a tube **118,** which is used to deliver a device **120** according to a further aspect of the present invention. The device **120** may be captured by a delivery tool **122** through the use of a ring or other fixation feature **124** mounted on the repair device **120.**

FIG. 23 shows a delivery method similar to that depicted in FIG. 22, with the exception that the tube **118A** has a reduced diameter so that it may enter into the sub-annular space of the disc **114** through the aperture or rent.

Turning to FIG. 25, according to a further aspect of the present invention, the delivery of the device **120** through the delivery tube **118** or **118A** may be facilitated by folding the arms or lateral extensions **128, 130** of the device to fit within the lumen of the tube **118** or **118A** so that the stent or device **120** is introduced in a collapsed configuration. The device **120** is moved through the lumen of the tubes **118** or **118A** through the use of delivery tool **122.** FIG. 25 shows the arms deflected in a distal, or forward direction for insertion into the delivery tube **118** or **118A** while FIG. 24 shows the arms **128, 130** deflected into a proximal position. FIG. 26 shows the device **120** curled so that one arm **128** is projecting distally, or in a forward direction, and the other arm **130** is projecting proximally, or in a rearward direction. Because the lateral extent of the device is relatively flexible, whether the device is of natural or synthetic material, other collapsible configurations consistent with the intent of this invention are also possible, including twisting, balling, crushing, etc.

FIG. 27 shows the device **120** having a series of peripheral barb structures typified by barb **132** located at the edges. In operation, these barbs may be forced into the annulus fibrosus as seen in connection with FIG. 28. Barb placement can be anywhere on the device **120** provided that at least some number of barbs are likely to find annulus fibrosus tissue to anchor in during placement. For a simple aperture or rent, placement on the periphery of the device body is a reasonable choice, but for complex tears, it may be desirable to place a plurality of barbs on the device not knowing in advance which barbs will find tissue to anchor in during placement.

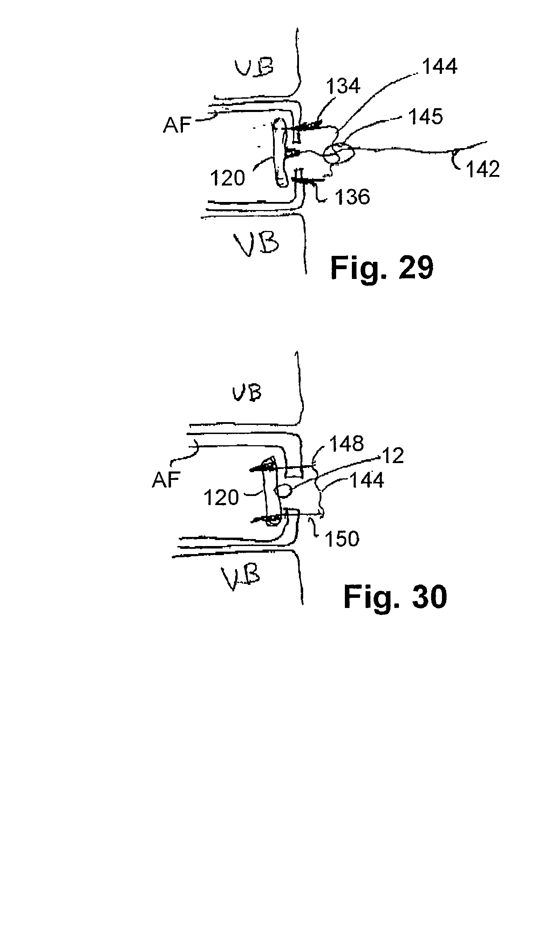

FIG. 29 shows an alternative fixation strategy where a pair of barbs **134** and **136** are plunged into the annulus fibrosus from the exterior of the annulus while the device **120** is retained in the sub-annular space by means of a tether **142.** Although there are a wide variety of fixation devices in this particular example, a tether **142** may be knotted **145** with the band **144** holding the barbs **134** and **136** together to fix the device in the sub-annular space. The knot is shown in an uncinched position to clarify the relationship between the tether **142** and the bands **144**. Using this approach, the device can be maintained in a subannular position by the barbed bands while the tether knot is cinched, advantageously simultaneously reapproximating the annulus to close the aperture while drawing the device into sealing, bridging engagement with the subannular wall of the annulus fibrosus.

FIG. 30 shows an alternative fixation strategy where the barbs **148** and **150** are sufficiently long that they can pierce the body of the device **120** and extend all the way through the annulus fibrosus into the device **120.** In this configuration, the band **144** connecting the barbs **148** and **150** may be tightened to gently restrain and position the device **120** in the sub-annular space, or tightened with greater force to reapproximate the aperture or rent.

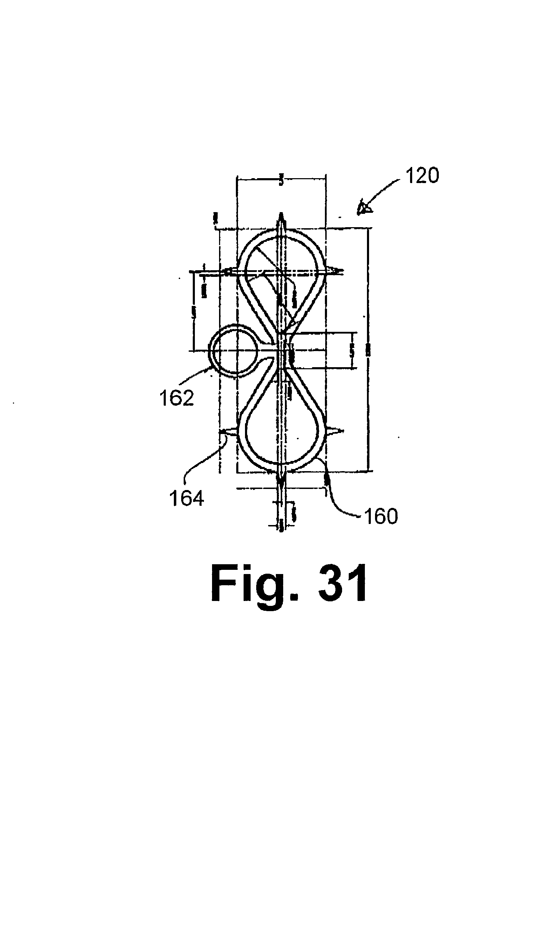

FIG. 31 shows a still further illustrative embodiment according to another aspect of the present invention. In this embodiment, a metal substrate **160** is incorporated into the device **120.** This piece can be machined from flat stock and includes the loop **162** as well as barbs typified by barb **164.** When formed in to the device **120** the structure shown in FIG. 31 is used in a manner analogous to FIG. 27 and FIG. 28.

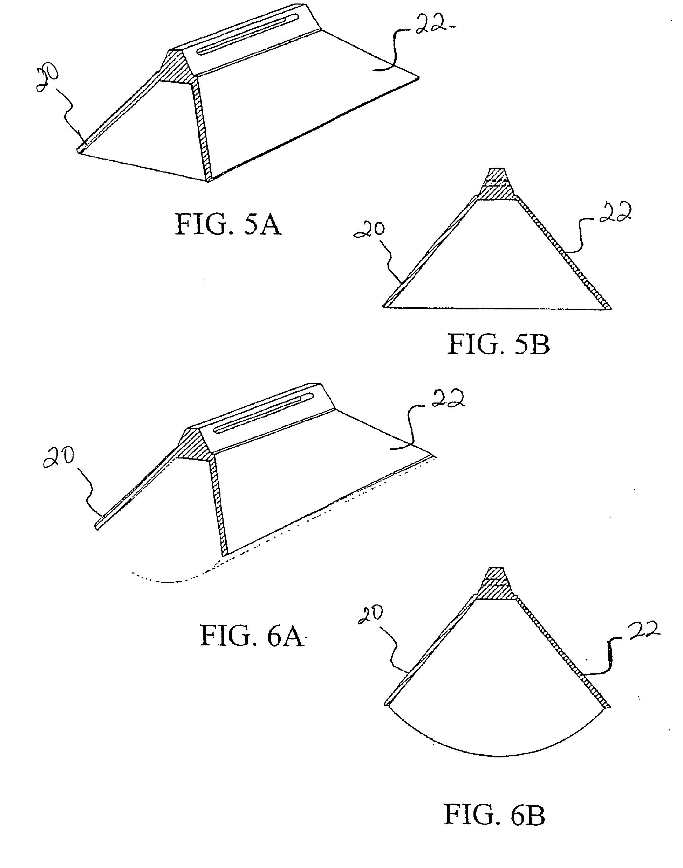

Stents can expand to be planar, for example as shown hereinabove in FIGs 4, 8, 9, 11 and 12, or they can expand to be three-dimensional as shown hereinabove in FIGs. 5 and 10. FIGs 34-36 depict a further three dimensional patch/stent using an autograft formed of fascial tissue. FIG 34 shows the superior vertebral body **202** and the inferior vertebral body **204** surrounding a disc having an annulus fibrosus **206** and nucleus pulposus **203** in the subannular space. According to this illustrative embodiment of the invention, a suture **210** is passed from outside the annulus through the wall of the annulus on one side of an aperture **208** and into the subannular space as shown. The suture is then passed back out through the annular wall on an opposing side of the aperture **208** leaving a loop or sling **212** of suture in the subannular space. As shown in the posterior view on the right side of FIG. 34, more than one suture can be applied. Turning to FIG. 35, a fascial autograft **214** is then inserted through the aperture **208** into the subannular space using, for example, forceps **216.** FIG. 36 shows the fascial stent/patch **214** fully inserted into the subannular space within the suture sling **212.** The closure of the aperture is accomplished simultaneously with pulling the autograft **214** toward the annular wall as shown in FIG. 37. The suture **210** can be cinched **218** or tied to maintain the closure and the fixation of the patch/stent.

Patches can be folded and expanded in a single plane or in three dimensions. As shown in FIGs. 24-25 and 41 for example, collapsing the patch can be accomplished laterally, whether the device is a single material or composite. Other embodiments, such as that shown in FIG. 1 can collapse vertically , and still others such as that shown in FIG. 26, longitudinally. Others can collapse in three dimensions, such as those shown in FIGs. 13-15 and 36. Devices which expand in three dimensions can be packaged in a restraining jacket, such as a gelatine shell or "gelcap" for example, or a mesh of biosorbable or dissolvable material, that would allow for facile placement and subsequent expansion.

Patches can also be constructed of a single component, as shown for example in Fig. 36, made of autograft or a synthetic material such as Dacron, or for example where the stent is a gelcap. They can be made of multiple components. An exemplary stent (not shown) can be made from a polymeric material, for example silicone rubber, which can be formed to have a natural unstressed shape, for example that of a "Bulb". A stylet or push-rod can, for example, be inserted on the inside of the bulb to stretch the bulb into a second shape which is thinner and elongated. The second shape is sufficient to place within the aperture in the annulus. Upon placement of the device within the sub-annular space, the push-rod is removed and the bulb assumes it natural, unstressed state, assuming a larger dimension within the sub-annular space. Although silicone is used in this example, other metallic constructs could also be envisioned such as a Nitinol braided device that has a natural unstressed shape and assumes a second shape under tension for the delivery of the device. It is also contemplated that the opposite scenario can also accomplish the similar objective. In this alternative embodiment, the device can have a first configuration that is unstressed and elongated and assumes a second, larger configuration (bulb) under stress. In this embodiment, a portion of the stylet or rod that is used to mechanically activate the device would be left behind to hold the expansion element in its stressed configuration.

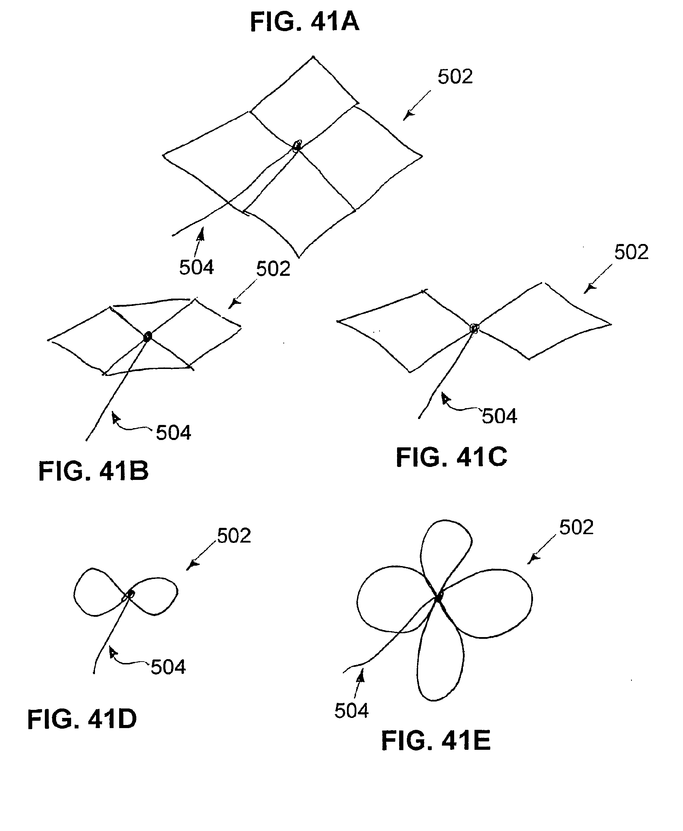

Multiple components could include a frame to help with expansion of the device and a covering to obtain biocompatibility and tissue ingrowth. Examples of different frame configurations might include an expandable "Butterfly" or "Figure-8" configuration that could be constructed of wire material, such as Nitinol or multiple wires. Exemplary embodiments showing frame members **502** are depicted in FIG. 41A-E. Of course, other configurations such as diamonds or other rounded or polygonal shapes can be used. The diamond frame is a construct that takes a first form that is smaller and expands to a larger frame. The diamond elements could be constructed from a single wire or from multiple wires. Alternatively, the members could be constructed of elements that are moveable fixed at each of the ends to allow expansion. A tether or attachment device **504** is also depicted, which may be a suture, a wire, a screw, or other attachment means known in the art.

The frame could be cut from a single material, such as flat stock Nitinol to accomplish the same objective, as shown for example in FIG. 31. Such shapes can be cut from flat stock using known methods, for example, laser cutting. A heat forming step could also be employed, as known in the art, to form barbs **132** in a shape that passes out of the flat plane of the stock material, as shown in FIG. 27 for example.

Another frame configuration, also not shown, is that of a spiral or coil. The "Coil" design can be, for example, a spring steel or other biocompatible material that is wrapped to a first "wound" smaller configuration and expands to a larger unwrapped, unwound configuration.

Depending on the size of the openings in the frames described above, each of these concepts may or may not have a covering over them in order to assure that the nucleus does not re-extrude from the intervertebral disc space after placement of the device, as well as to serve as substrate for the surrounding tissue to naturally incorporate the device. Coverings might include ePTFE, polyester, silicone, or other biocompatible materials. Coverings could also include natural materials such as collagen, cellulose, autograft, xenograft, allograft or similar materials. The covering could also be biodegradable in nature, such as polyvinyl lactic acid.

Frames that are not covered may be permeable, such as a patch that is porous and allow for normal movement of fluids and nutrients through the patch into and out of the annular ring while maintaining nucleus fragments larger than the porosity of the stent/patch within the subannular space. Depending on the material that the frame is constructed, a surface finish may be added to promote tissue ingrowth into the patch. For example, a titanium sputtering of the device may allow it to be more easily incorporated within the disc space. Alternatively, a NiTi or tantalum foam could be added to the outer surface of the patch to promote tissue ingrowth.

It is understood that there can be a variety of device designs of patches to accomplish the expansion of a device from a first configuration, to a second configuration to occupy the sub-annular space and reduce re-extrusion of the nucleus. The following device concepts are further discussed for additional embodiments of a device and/or system for the repair of an intervertebral disc annulus.

As mentioned hereinabove, the stent/patch according to the present invention may comprise a mass of fascial autograft, and that autograft may be contained in a covering of material to form what will be referred to herein as a "bag". Of course, this term is used not necessarily to connote a five-sided closed container so much as to denote the notion of flexibly surrounding the volume of a patch/stent material so that it can be manipulated in space.

In the most simplistic form, a prefabricated device of sutures could be used to form the "sling" to hold the fascial implant as discussed above. The advantage of this design over simple placement of sutures to hold the autograft is better containment and control of the autograft during and after implantation. The "sling" or a "bag" surrounds the fascial autograft to hold it in place. It is contemplated that other materials, such as a polyester mesh, could be used instead of the fascial autograft.

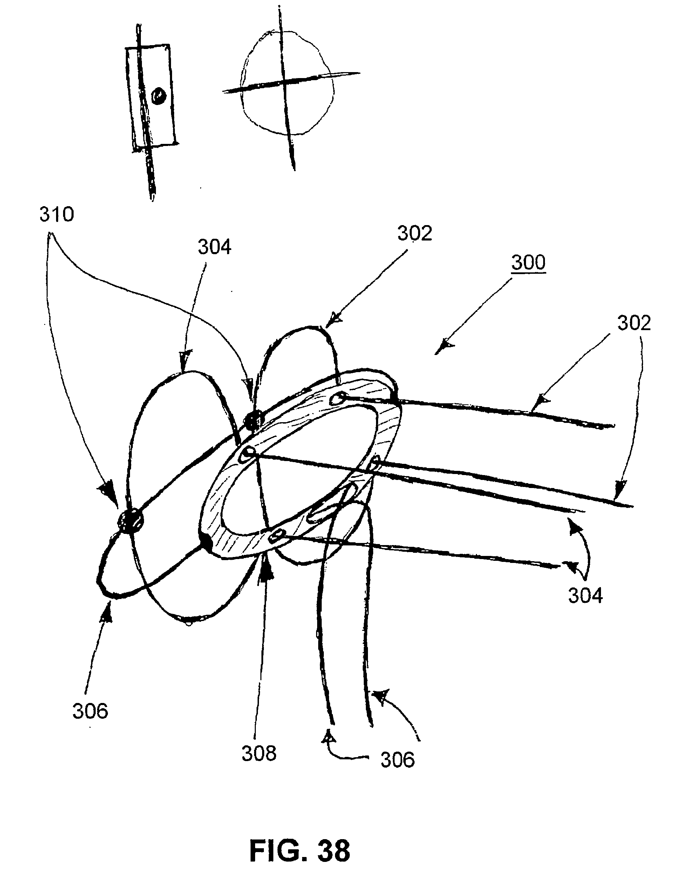

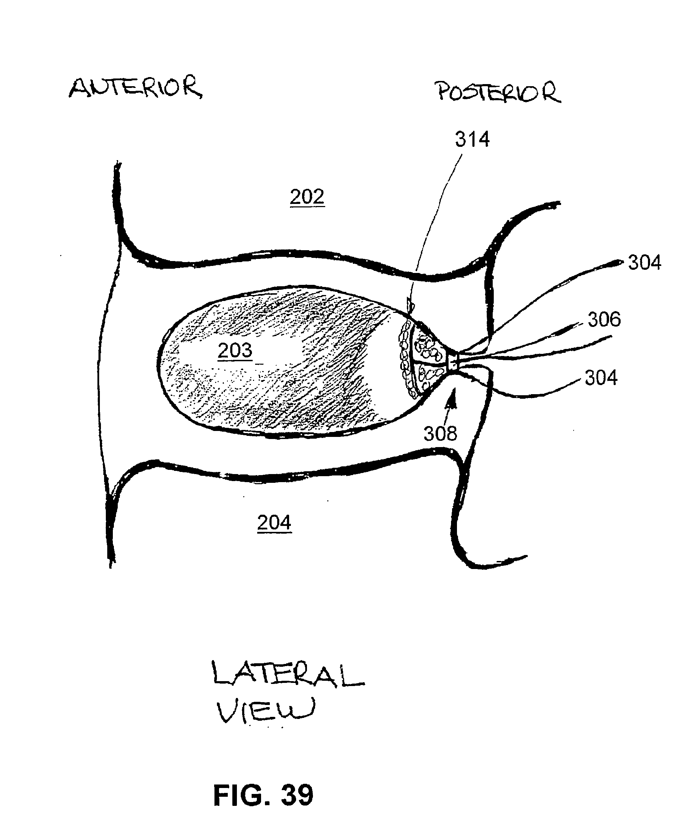

Figure 38 shows an example of a pre-fabricated sling **300.** There are three sutures used in this example, **302, 304,** and **306,** although there could be more or less sutures as would be understood by one of ordinary skill in the art. A collar member **308** has apertures or other features for attaching to the sutures. In this example, the third suture **306** passes along or within the collar **308** to form a loop extending from the lateral extent of the collar **308.** The first and second sutures **302, 304** form loops from the superior and inferior extents of the collar **308.** Intersections **310** can secure the loops to each other with small loops or knots in the sutures, small fabric attachment pieces, or by small preformed devices resembling grommets placed on the suture to aid in securement. Other knot tying techniques known in the art can also be employed. Turning to FIG. 39, the collar is depicted within the subannular space where the loops surround a fascial autograft **314** which by pulling proximally the sutures **302, 304, 306** the graft is collapsed into contact with the annular wall in a sealing manner. The sutures can be made of known materials, e.g., biodegradable, bioabsorbable or bioresorbable Vicryl or biocompatible nylon. The collar can be made of a fabric material, *e.g.,* polyester. During placement, one end of some or each suture can be passed through the inferior wall of the annulus and the other end can be passed through the superior wall surrounding the aperture. After the placement of the sling into the wall of the annulus, the fascial autograft is placed within the sling. The sutures are tightened to bring the tissues together and also to help reappoximate the aperture, as the collar size will be selected based on the surgeon's judgment according to the degree of reapproximation desired.

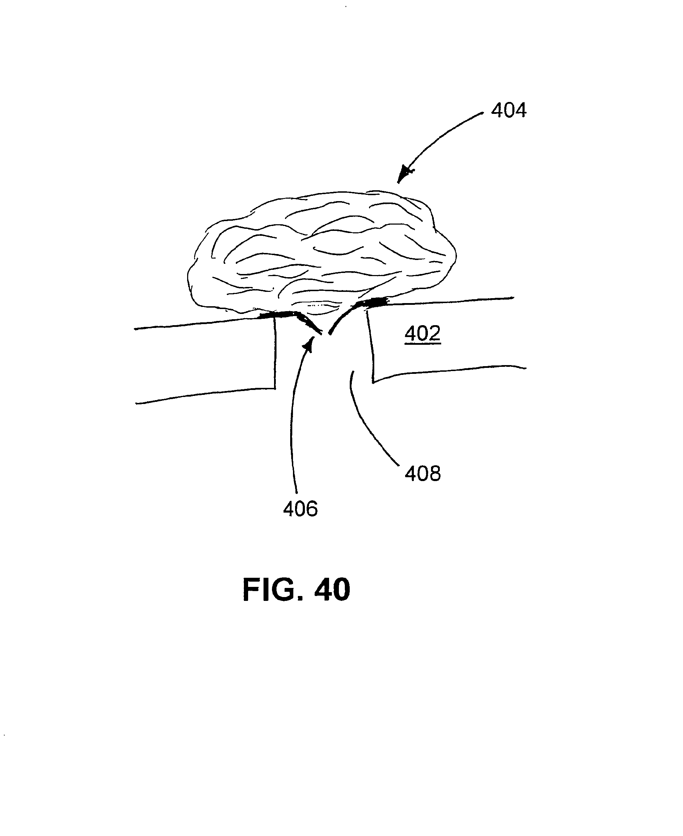

Other constructions can also be used to accomplish the same objective, such as a "bag" **404** formed of expandable PTFE as shown in Fig. 40. The bag is placed through an aperture in the annulus **402.** Additionally, a one way seal **406** can be positioned behind the aperture **408.** Suturing techniques for introducing cardiac valves could be employed to place the seal. It is understood that there could be multiple constructs to accomplish the same objective and this is only given as an example.

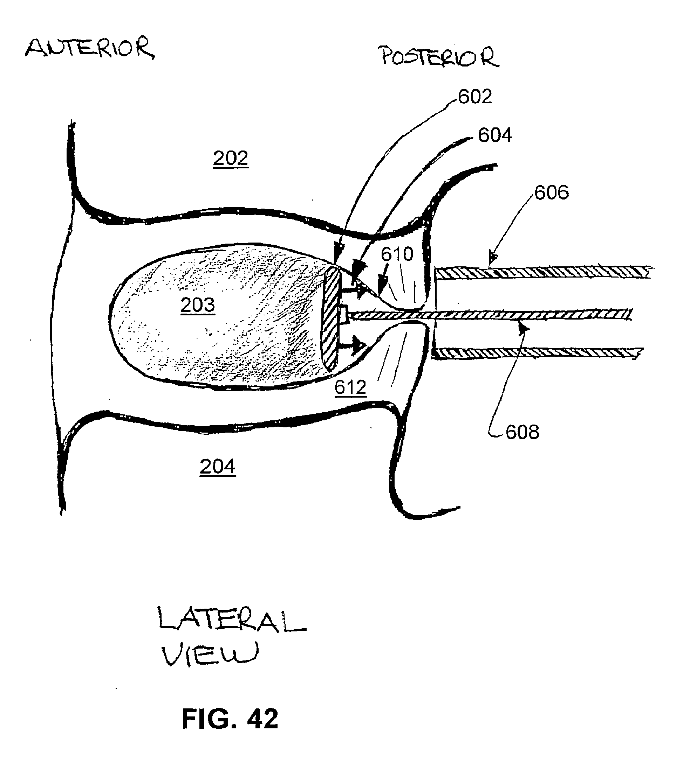

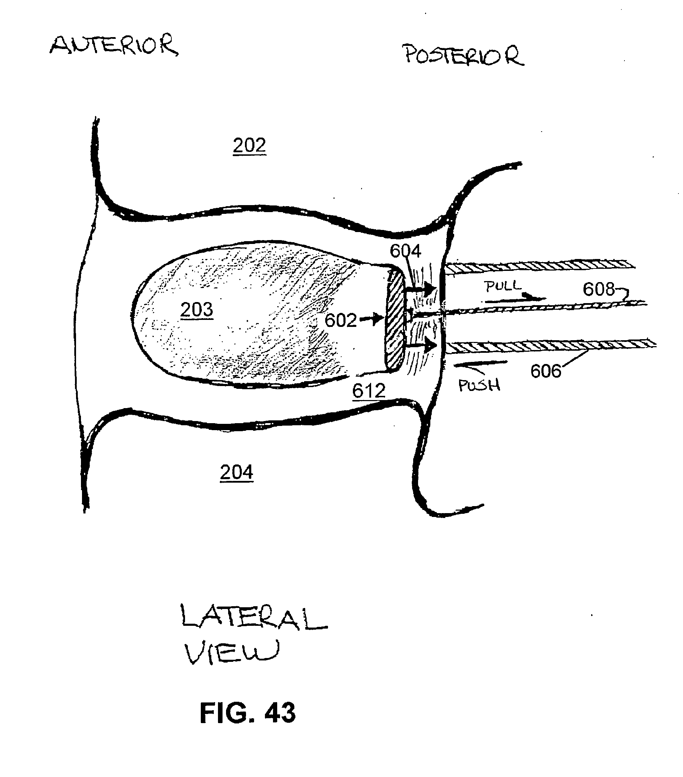

The are a variety of ways to affix the device to the sub-annular wall of the annulus in addition to those discussed hereinabove. The following exemplary embodiments are introduced here to provide inventive illustrations of the types of techniques that can be employed to reduce the time and skill required to affix the patch to the annulus, versus suturing and tying a knot. Discussed hereinabove is the use of sutures, staples and other fixation devices, such as those passed through slot **18** to affix the patch to the annulus as shown in FIG. 1. FIG. 20 also depicts the use of "barbs" on the surface of the stent to facilitate fixation to the annulus. In a simple example, as shown in FIG. 20, a patch/stent could be compressed, passed through a guide tube such as tubes **18, 18A** shown in FIGs. 22 and 23, and expanded within the sub-annular space. As shown in FIG. 42, the expanded patch **602** is shown having barbs **604,** along with detachable delivery tool **608** and guide tube **606.** Once expanded, barbs **604** on the outer surface of patch **602** can be used to fix the patch into the inner wall **610** of the annulus **612** by pulling the patch back proximally, into the sub-annular wall **610,** and pushing forward distally on the guide tube **606,** thus driving the barbs **604** into the annulus and drawing the inner and outer tissues of the annulus together and reapproximating the disc on either side of the aperture, as shown in FIG. 43. After the placement of the patch, the delivery tool and guide tube are removed.

The advantage of this design described above is that it requires very little time and skill to place and secure the patch to the annulus while also drawing the tissues together.

Materials of the patch could be similar to materials discussed hereinabove. Anchoring barbs could be made of a biocompatible material, for example a metallic material (e.g., NiTi alloy, Stainless steel, Titanium), or a polymeric material (e.g., polypropylene, polyethylene, polyurethane). Anchoring barbs could also be a biodegradable/bioabsorbable material, such as a polyglycolic acid (PGA), a polylevolactic acid (PPLA), a polydioxanone (PDA) or for example a racemic polylactic acid (PDLLA). If the barbs included a biodegradable/bioabsorbable material, it is anticipated that the barbs might have sufficient holding strength for a sufficient period of time to allow the patch to be incorporated into the annulus during the healing process. The advantage of having the anchoring barb of FIGs. 42 and 43 being biodegradable/bioabsorbable is that after the incorporation of the patch into the annulus there may be no need for the barbs to provide fixation. However, barbs pointing toward the outer surface of the annulus could pose a long term risk of penetration out of the annulus due to migration, and potentially impinging on the nerve root and spinal canal. Biodegradable/bioabsorbable barbs address and advantageously reduce any long-term risk in this regard.

It is also possible that the barbs could be made of both a biocompatible component and a biodegradable/bioabsorbable component. For example, the very tip of the barb could be made of a biodegradable material. The barb could penetrate the annulus wall with a rather sharp point, but after degradation the point of the barb would become dull. In this embodiment, the point would no longer induce continued scar formation after the patch has been incorporated, nor pose a risk of penetrating out of the annulus onto the nerve root.

Another fixation means includes the passing of "anchoring bands" into the wall of the annulus, vertebral bodies (superior, inferior, or both), or the Sharpey's Fibers (collagenous fibers between the junction of the annular fibers and vertebral bodies). In the following example of anchors, the barbs or bands are affixed to the annulus/vertebral bodies/Sharpey's fibers. Another element, for example a suture, cinch line, or a staple is utilized to attach the anchor bands to the patch, and thus hold the patch in proximity to the inner wall of the annulus. In addition, these bands may re-approximate the tissues at the aperture.

Revisiting one example of using barbs to anchor the device is shown in FIG. 9, described hereinabove. Barbs or bone anchor screws **50** and **52** are passed into the superior and inferior vertebral bodies **54** and **56,** respectively. Superiorly, suture **40** is passed through the outer wall of the annulus, to the sub-annular space. The suture is then passed through the eyelet **53** of bone anchor screw **52** and then passed through the wall of the annulus from the sub-annular space to the outer wall of the annulus. The inferior end of the suture is similarly passed through the annulus, eyelet of the bone anchor, and back through the wall of the annulus. Both ends of suture **40** are tightened and tied. The advantage of this concept is that it allows for fixation of the device to a surface that is known to be present in all discectomy procedures - the vertebral bodies. Whereas, it is possible, depending on the location and size of a natural rent that there may not be sufficient annulus accessible to fixate the device directly to the annulus. In addition to providing a location for fixation, anchoring into the vertebral bodies may provide a more stable anchor surface.

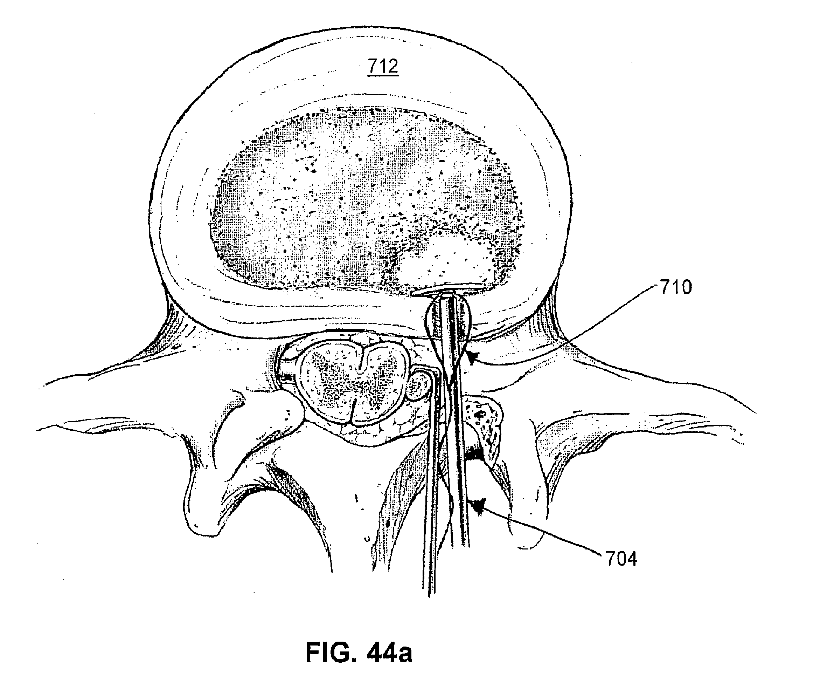

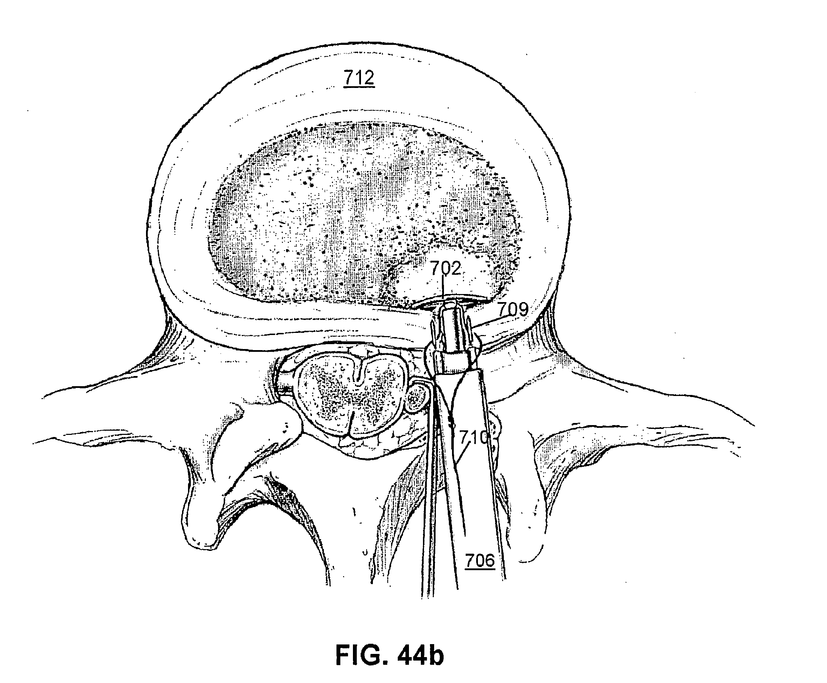

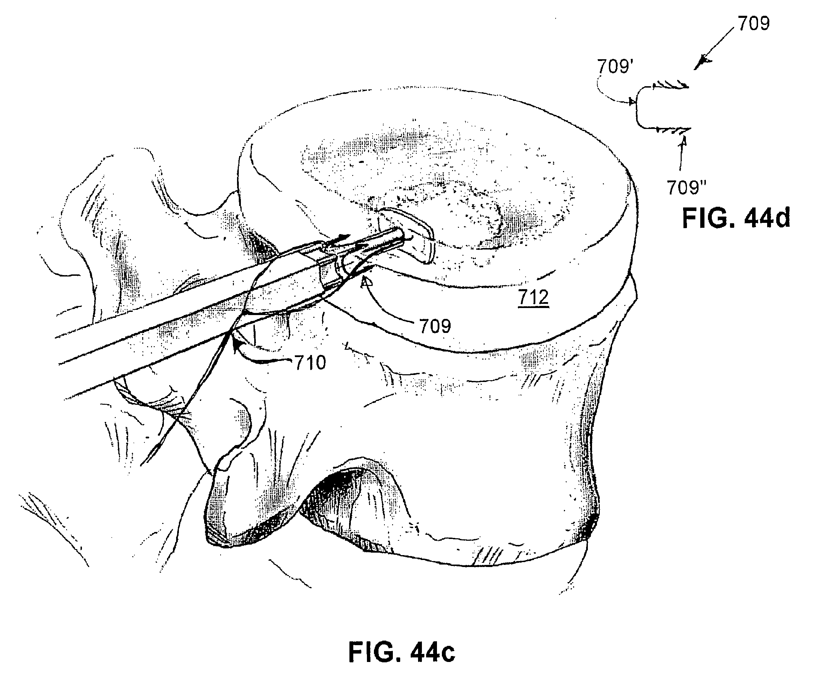

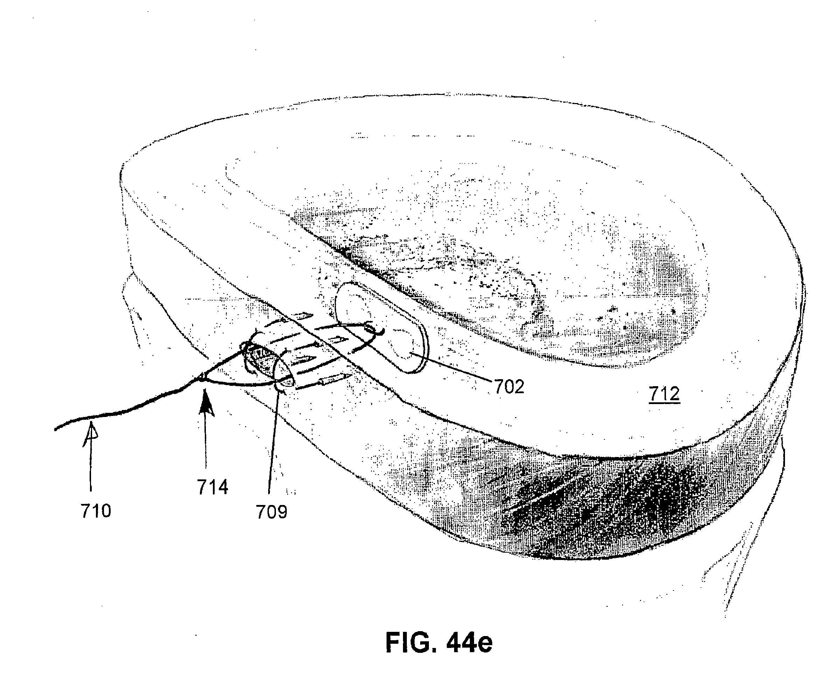

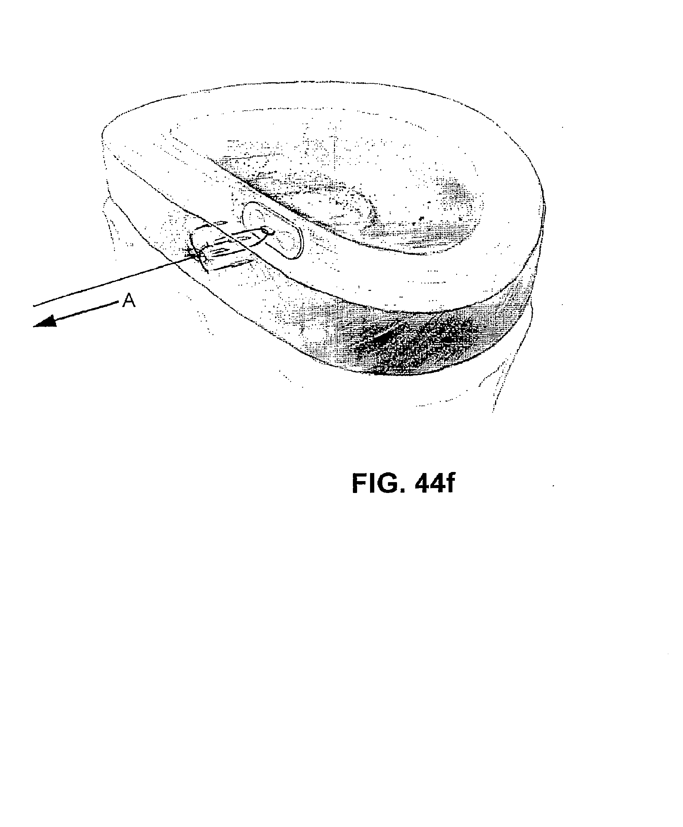

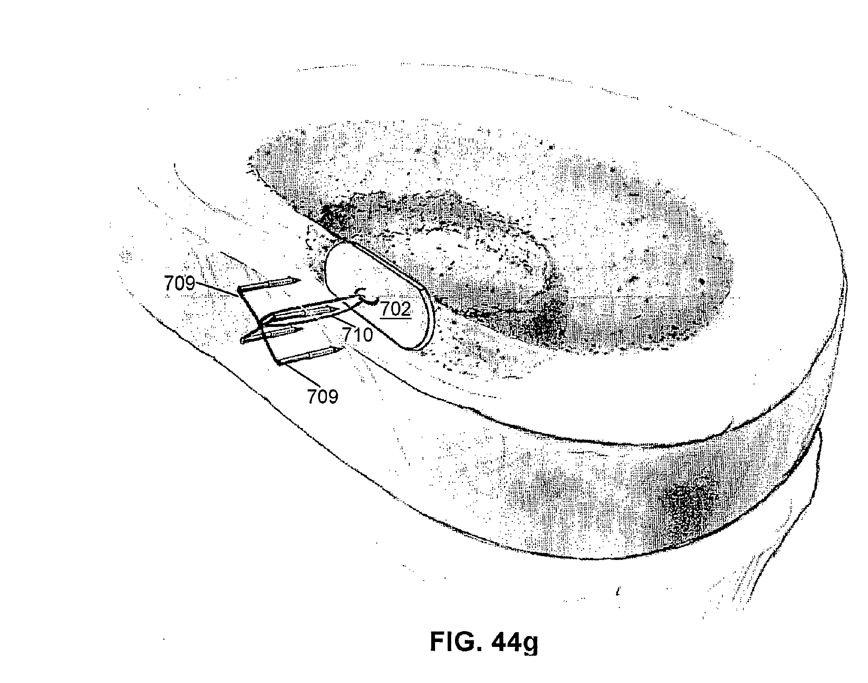

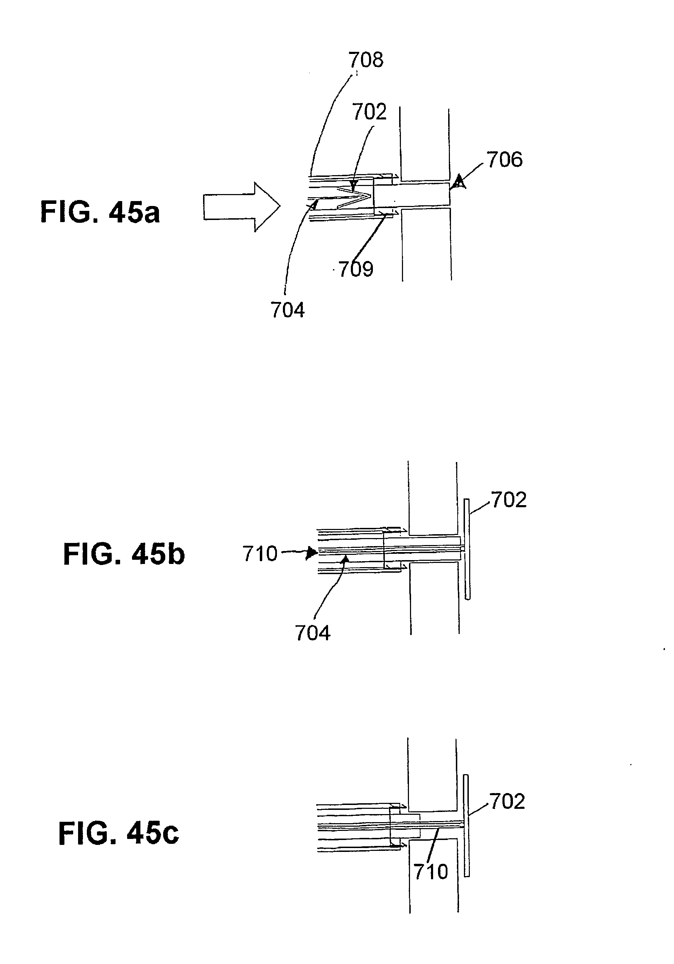

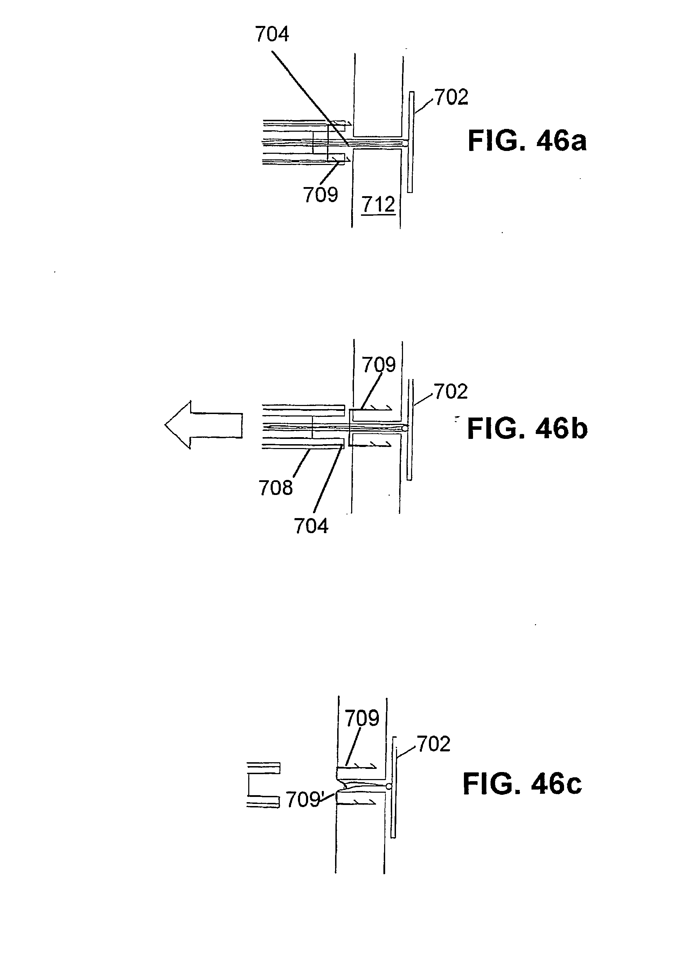

Another example of fixating the device to inner wall of the annulus is shown in FIG. 29, and is further illustrated by FIGs 44-47. As discussed hereinabove, with reference to FIGs. 22-30, a patch **120** is placed with a delivery tool **122,** through the inner lumen of a guide tube **118,** into the sub-annular space and then expanded. This step can also be seen in FIGs. 45 and 46, where a patch **702** is folded and passed through a guide tube **706** and is held by a delivery tool **704.** Also shown is a anchor band or staple 709 and an anchor band delivery device **708.** Within the guide tube, or within the delivery tool, there is a suture line or cinch line **710** that is attached to the center of the patch **702.** This can be seen in FIG. 44a with the guide tube **706** removed. As seen in FIGs. 45C and 46A, the guide tube **706** is retracted after the patch **702** has been expanded and deployed. Next, an anchor band delivery tool **708** is used to deliver one or more "bands" **709** onto the outer surface of the annulus. These are intended to be anchored into the wall of the annulus with barb shapes that do not allow for the barbs to be pulled back through the annulus. The anchor bands resemble a construction of a "staple". The bands could actually be constructed by connecting two barbed elements with, for example, a suture between the two barbed elements. The barbs and the connection band between the barbs could be constructed of the same material or of different materials. For example, the barbed part of the anchor band could be a biodegradable/bioabsorbable material (such as polyglycolic acid) or could be constructed of a metallic or polymeric biocompatible material (e.g., titanium, NiTi alloy, stainless steel, polyurethane, polypropylene). In addition, the band that connects these barbs can be constructed of materials that are similar to the barbs, or different materials. For example, the connection band could be a biodegradable/bioabsorbable suture, such as Vicryl, or a biocompatible material such as polypropylene. In addition, it is possible that these elements are constructed from multiple materials to accomplish the objective of anchoring into the annulus and providing for a fixation site to draw the patch within proximity of the sub-annular wall.

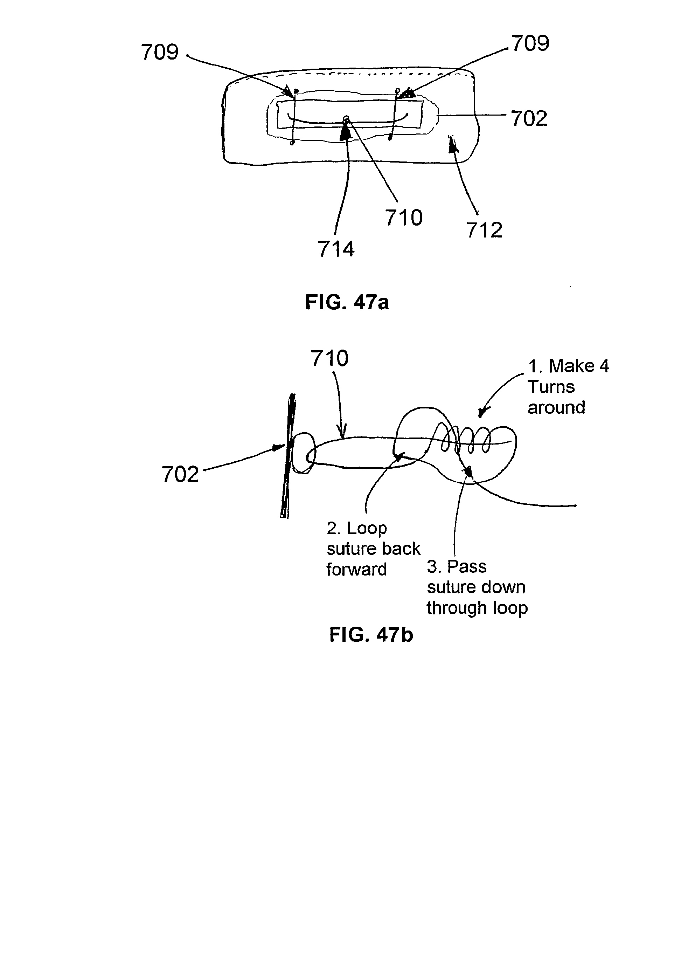

FIGs. 44B and 44C show the placement of the anchor bands **709** into the annulus **712** with the anchor band delivery tool **708.** FIGs. 46A and 46B schematically show the placement of the anchor bands **709** into the wall of the annulus **712** and the retraction of the anchor band delivery device **708,** with the patch delivery tool **704** still in place. FIG. 44D depicts a representative anchor band **709,** having a pair of stainless steel barbs **709"** connected by a suture **709'**. FIG. 44E shows the patch **702,** anchor bands **709,** and cinch line or suture **710** with the delivery tools removed, prior to drawing the patch and the tissues of the annulus together. In this embodiment there is a pre-fabricated knot **714** on the cinch line, which is described further in Fig. 47B, although other knots are possible. FIG 47a also shows a posterior view of the patching of the annulus with this device with knot **714.** In this stent/patch **702** a pair of loops of 7mm suture **709** are shown, which engage the cinch line and slip knot. These suture loops connect to the barbs directly, as in FIG. 44, or loop to surgical staples, or are placed directly into the annulus. The presence of a pre-fabricated knot on the cinch line makes the process of repairing quicker since there is no need to tie a knot. It also facilitates drawing the tissues together. The use of the cinch line and a pre-fabricated knot can be placed by, for example, an external tube such as a knot pusher. Fig. 44E is similar to the FIG. 29 described hereinabove prior to "tying" the knot **145.** Fig. 44F shows the drawing of the patch and the annular tissues together by pulling on the suture in the direction "A" indicated by the arrow. In this case, the Knot Pusher has been removed from the cinch line **710.** The suture **710** is drawn proximally to draw the patch **702** into engagement with the inner wall of the annulus to seal the aperture from within, as well as draw the walls of the annulus together to reapproximate the annular aperture. FIG. 46C and FIG. 44G show the cinch line suture **710** tied and drawing the annular tissues together, after the excess suture line has been cut. It is also apparent from this device, fixation and delivery system that the outer surfaces of the aperture are also drawn together for re-approximation.

The cinching of the bands and the patch also allows for taking-up the slack that allows for the accommodation of varying sizes. For example, the thickness of the annular wall surrounding the aperture can vary from 1 mm up to 10 mm. Therefore, if the anchor bands have a set length, this design with an cinch line accommodates different dimensions of the thickness of the wall of the annulus by drawing the "slack" of the bands together within the aperture.

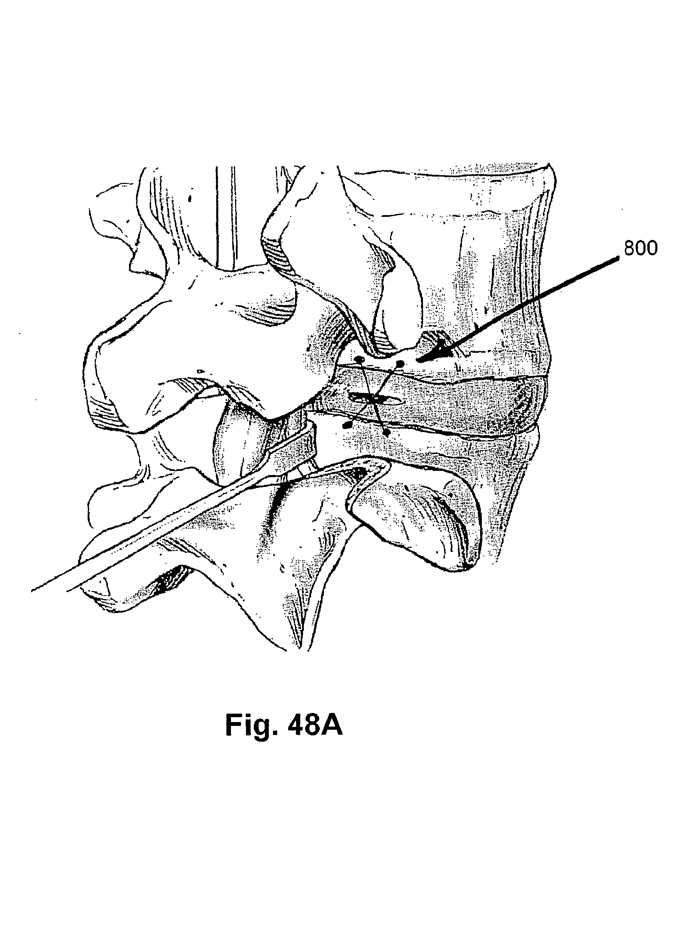

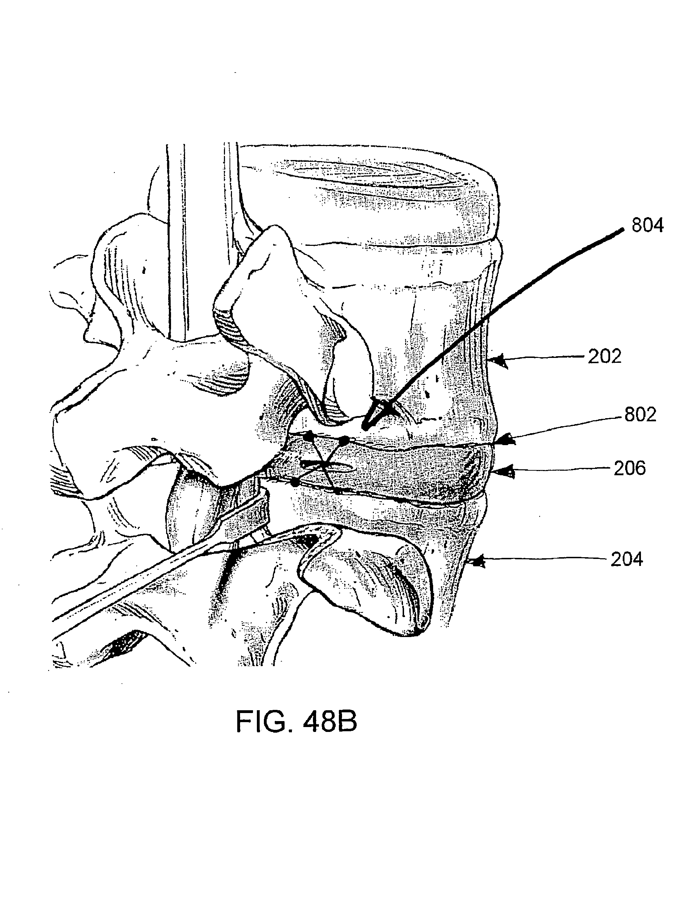

Although it has been described here as patch placement that involves two lateral anchor bands with a suture to draw the patch, bands and tissues together, one or more bands could be used and two bands is only an example. Furthermore, the anchor bands were placed with the barbs in a superior-inferior fashion. One skilled in the art would recognize that these could be placed at different locations surrounding the aperture. Moreover, although it was described that the bands are placed into the annulus, these anchor bands could also be placed in the vertebral bodies as shown in FIG. 48A generally at **800,** or the Sharpey's Fibers **802,** as shown in FIG. 48B generally at **804.**

Although the patch depicted in the example above does not have barbs attached to the patch, it is also possible to have the barbs as described hereinabove to further promote the fixation of the patch to the inner wall of the annulus.

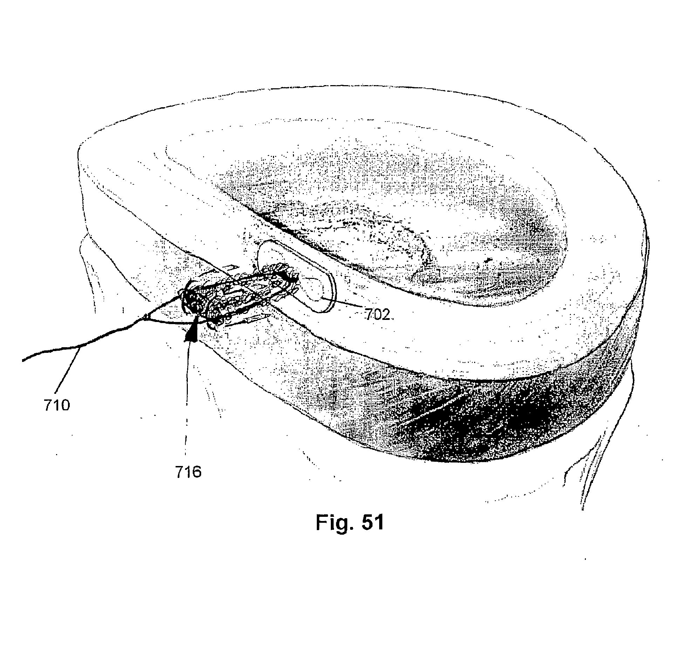

Finally, although the drawings depict an aperture that lends itself to re-approximating the tissues, it is conceivable that some apertures, whether natural or surgically made, may be relatively large and therefore might require the placement of additional material within the aperture to act as a scaffold for tissue in growth, between the patch on the inner wall of the annulus and the anchor bands located on the outer wall. An example of material to fill the aperture might include autograft para-spinal fascial tissue, xenograft, allograft, or other natural collagenous materials. The filler material could also be of a biocompatible material such as a Dacron material. FIG. 51 shows the illustrative filling of an aperture with implant material **716** prior to cinching the suture **710.**

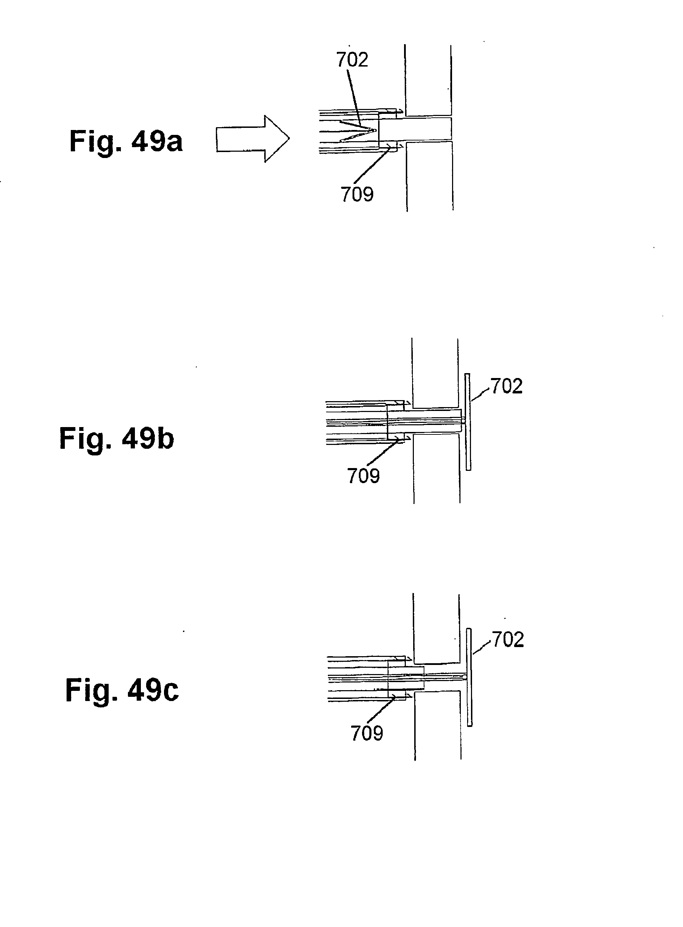

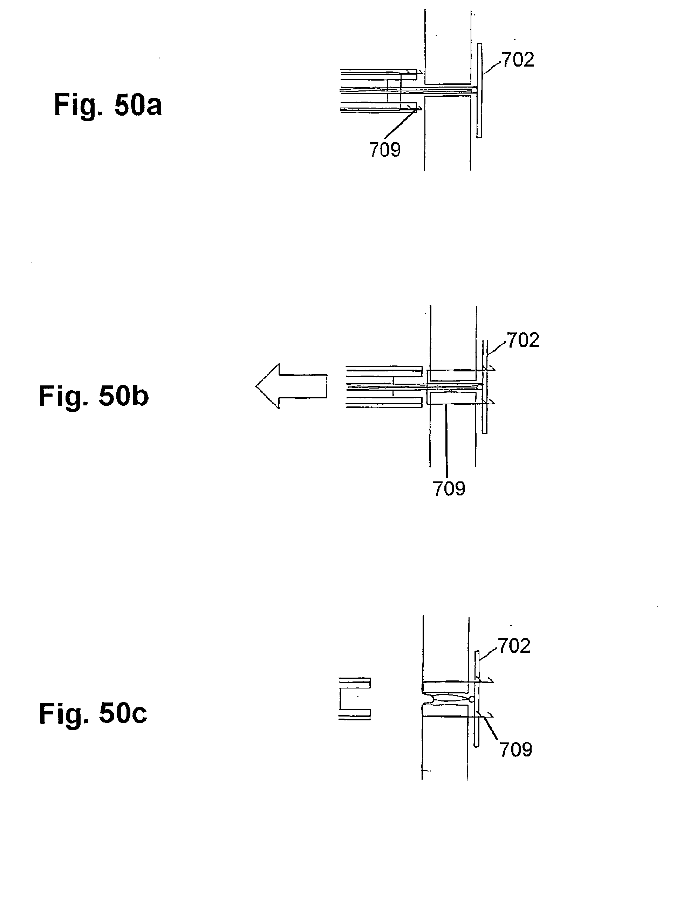

As an alternative embodiment of the present invention, the anchor bands **709** as described previously (anchor bands into annulus) could be sufficiently long enough to pass through the annulus and then through the patch. The barbs in this embodiment have an engaging involvement with the patch. This concept was previously discussed hereinabove in connection with FIG 30. Further illustration of such a system is schematically shown in FIGs. 49 and 50. Passing the barbs through the patch, in this embodiment, provides additional security and safety of reducing the possibility that the barbs may migrate after implantation. In this application of the invention, the suture cinch line may (FIG. 50) or may not (Fig. 30) be used in addition to the anchor bands to draw the tissues together and reduce tissue movement surrounding the aperture.

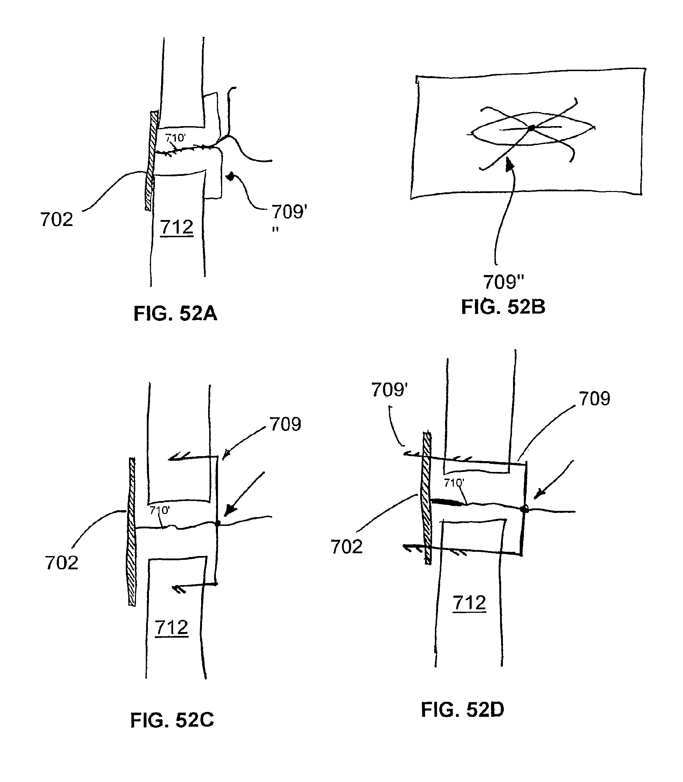

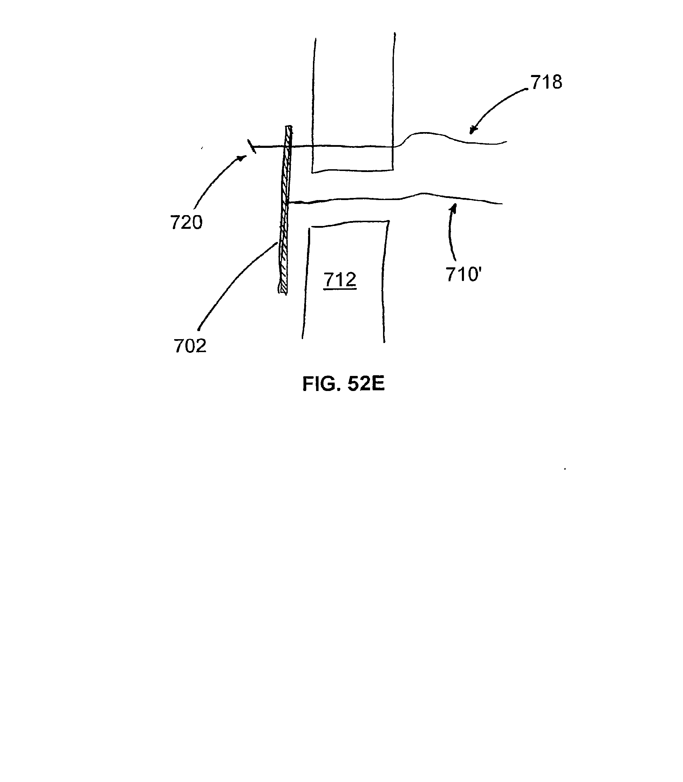

In addition, although the bands shown in Fig. 49 and 50 take the form of a "barb", they could as easily take a form of a simple T-barb **720,** as shown in FIG. 52E, or a C- type element wherein the object is to have irrevocable engagement with the patch device **702** after the penetration through the patch. A T-type attachment, when aligned longitudinally with the suture, passes through the patch. The T section then rotates to prevent the suture anchor from being pulled back through the patch. In another embodiment a "C' retainer made of a superelastic material may be attached to the end of the suture band. The C retainer is loaded into a needle wherein it is held straight. The needle is used to pass the C retainer and suture through the patch and deploy the retainer in a second configuration in the shape of a "C".

It is also foreseen within the scope of the invention that there may be patch designs which will accommodate the placement and securement of the anchor to the fabric that covers the frame of the patch. For example, a frame for a patch that is made out of metal such as Nitinol can provide for "windows". The device, covered with a mesh fabric, for example silicone or Dacron, would therefore allow the anchoring barbs to be passed through the "windows" in the frame of the patch. In this case, the barb can be secured to the patch in the fabric covering the frame.

Alternatively, the patch can be secured by passing barbs that engage the lattice of the patch frame. These embodiments of the invention illustrate designs in which the barbs engage with the vertical, horizontal or criss-crossed structures/members of the frame. In this case, the barbs would pass through the mesh or lattice of the frame and they would be unable to pass back out of the structure.

Although this discussion refers to "anchor bands" that are shown to be two anchors connected by a suture, it is also contemplated that single barbs with sutures are placed and the sutures' ends, at the outer surface of the annulus, are tied after placement through the patch.

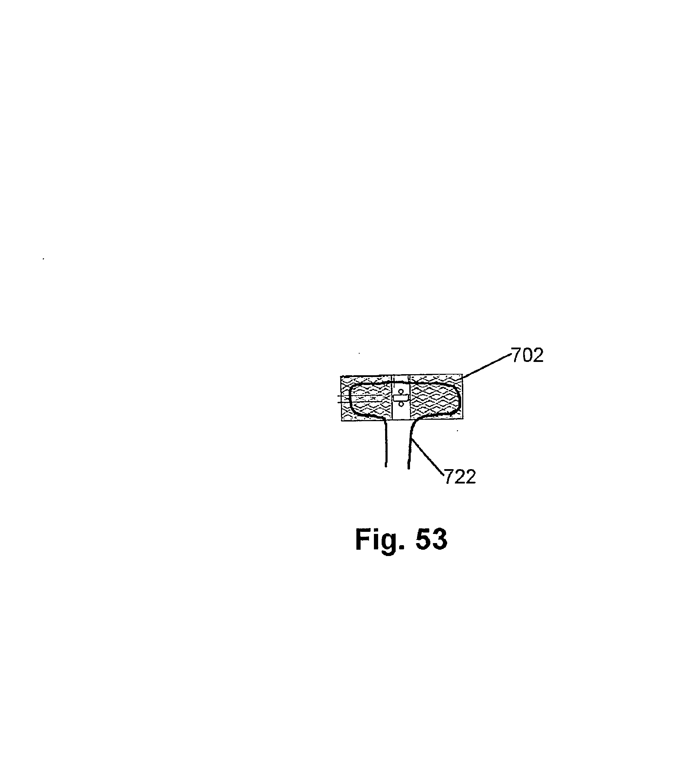

One objective in the designs discussed hereinabove is to provide a way to "pull up the slack" in a system to adjust the length of sutures and for anchor bands. According to the present invention, a technique referred to as the "Lasso Cinch Knot" was developed as a means to draw the anchor bands together with a suture cinch line that is incorporated into the patch design. Fig. 53 gives further description of the use of the Lasso embodiment. In essence, patch and frame constructs are used that incorporate the "barbs through the patch" design. Once the barbs have passed through the patch, an internal lasso **722** is drawn tight around the sutures of the anchor bands and thus draws the extra suture material within the patch. The internal lasso gathers the sutures of the bands, and as the lasso is tightened, it cinches together the sutures of the bands and therefore tightens them and eliminates slack, bringing the patch/stent into closer or tighter engagement with the annulus wall. The patch in FIG. 53 additionally provides for a diamond shape grid pattern, which advantageously provides a grid which will while allowing a probe or similar instrument to pass through with little resistance, provides resistance to a barb or other restraining feature on the instrument. The frame shown can be made from nitinol, and the locking and holding windows shown at the center of the figure would allow for rotation about the z-axis during placement. A slipknot technique using, for example a knot pusher, would aid in the loop pulling process by the lasso. The internal loop (lasso) can be tacked to the outside corners of the patch/stent, in order to hold the loop at the outer edges of the patch frame. When cinching the lasso knot, the loop can be pulled free from some or all of its tacked attachment points to the frame, to prevent deformation of the planar shape of the frame when cinching the lasso. As above, the frame can be a composite structure or sandwich formed with some type of mesh fabric. The proximal mesh fabric can be bonded fully to the patch frame, for example through the use of an adhesive, for instance a silicone. Adhesive, advantageously, can fill the interstices of the grid pattern while allowing for easy probe penetration and protection of the suture lines. Protection of the suture lines is advantageous when the lasso is used to pull and bunch a group of band sutures together.

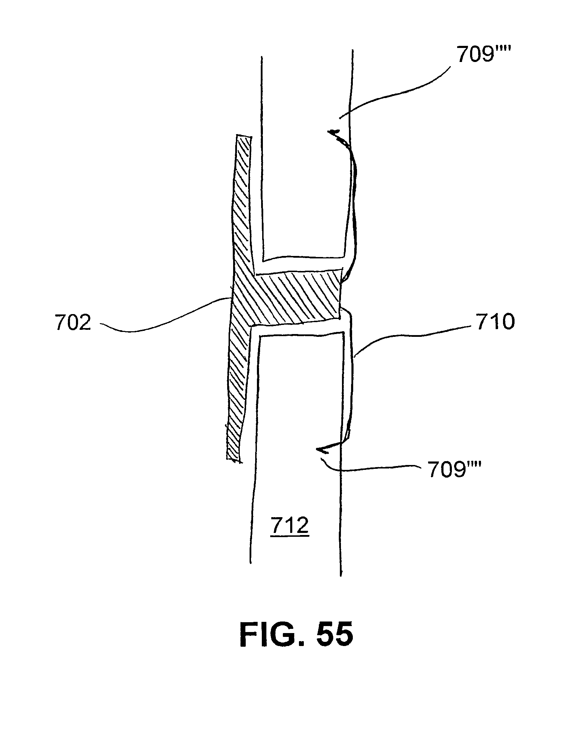

It is also contemplated within the scope of the present invention that sutures **710'** can be preattached directly to a stent/patch. As shown in FIG. 52A several separate barbs **709'"** into the annulus **712** can be directly attached to the patch **702.** Each "barb" of FIG. 52A can be independently placed into the annulus after the patch is deployed. This can be seen to be similar to the embodiment including barbs **709""** of FIG. 55.

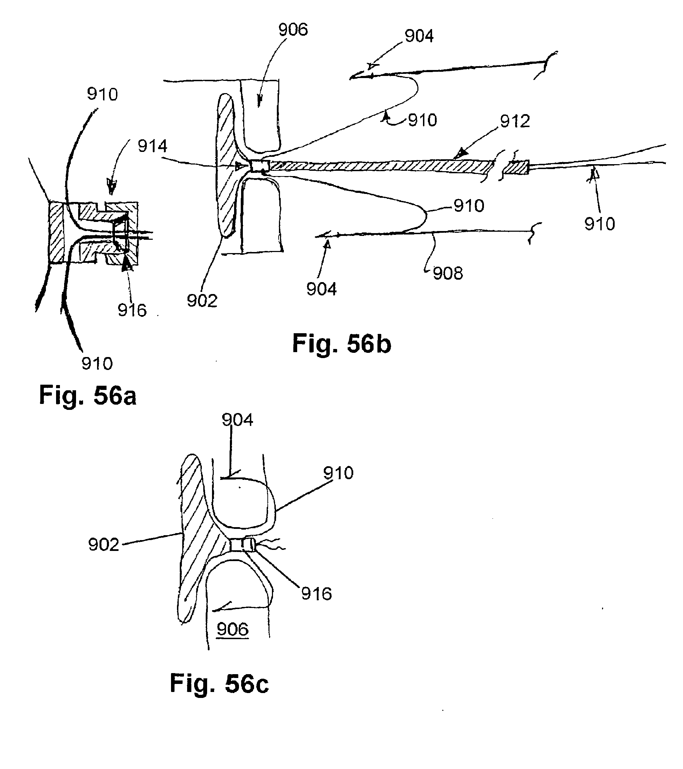

An alternative embodiment for securing a patch **902** and reapproximating a rent is providing each of the separate barbs with sutures having variable lengths as shown in FIG. 56. Each independent suture barb **904** is placed into the annulus **906** or into the patch **902** with the barb delivery tool **908.** After the placement, all of the suture lines **910** are drawn taught, by drawing on the free ends that exit the patch delivery tool **912.** A locking element **914** that uses a gasket **916** and threading mechanism is attached to the patch **902** and is used to tighten the gasket **916** around the distal ends of the sutures **910.** The patch delivery tool **912** is removed and the extra suture length is cut. It is also possible that the gasket mechanism could be a press-fit to accommodate the tightening of the sutures to the patch.

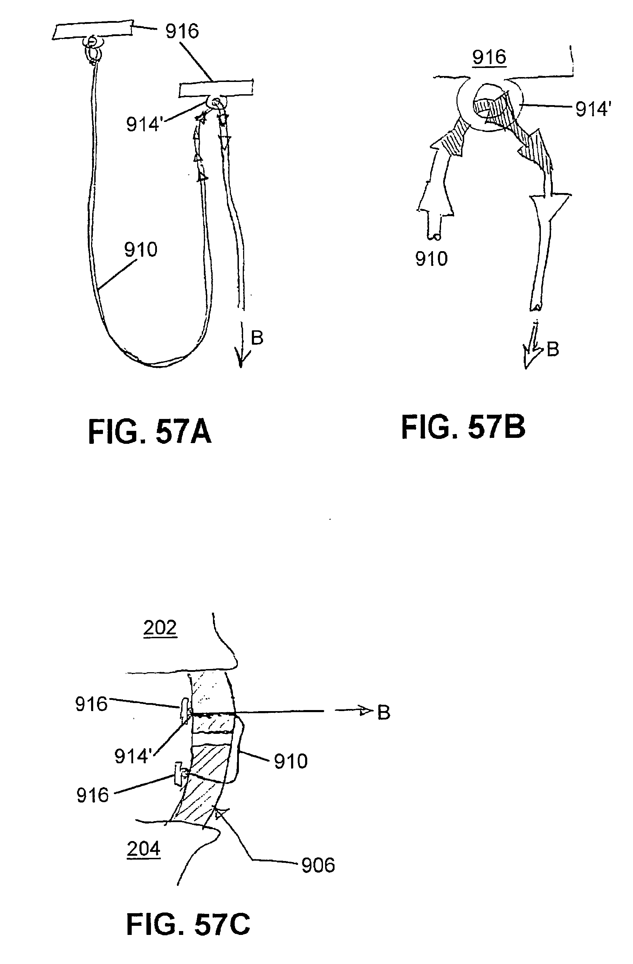

Alternatively, the locking mechanism can be as shown in FIG. 57, although in this case the engagement of the locking element **914'** takes part on the patch. Pulling the suture **910** in the direction of arrow **B** will tighten and lockingly hold in tension to aid in securement and reapproximation. The adjustable length suture band between the two anchors allows slack to be taken up between the anchors **916.** Two T-type anchors are illustratively shown in this example, but multiple anchors of differing configurations could be used. The locking features can be included on the feature band, as depicted here, and allow for substantially one-way locking engagement with the anchor members. This adjustability advantageously promotes for the accommodation of varying thickness of the annulus from patient to patient. The suture slack in this embodiment may be taken up to close the defect in the annulus and/or to shorten the band between anchors for a secondary cinching of multiple tensioned suture bands as described hereinabove.

The cinch line and the Lasso concepts in essence try to facilitate the re-approximation and drawing of tissues together in a fast and simple way. Other contemplated embodiments for "tension" elements include using an elastic coupler as a part of the anchor band used to fixate the device. The elastic coupler can be expanded for placement, and upon release, can draw tension to pull the tissues together. The coupler could be made of a biocompatible metal or polymer, or could be constructed of a biodegradable/bioabsorbable material.

Similarly, an alternative embodiment to cause tension within the device and draw the tissues together after placement of the anchor bands might include an elastic band or band with a spring which one end can be attached to the anchor bands and the other end attached to the patch. Alternatively, the anchor bands might, in and of themselves may be made of an elastic band between the barbs, or may contain a spring element between the barbs. Such an embodiment can be made to resemble a so-called "Bobber Spring." Again, it is contemplated that the elastic or resilient element could be made from a wide variety of metals, polymeric, or biodegradable/bioabsorbable material.

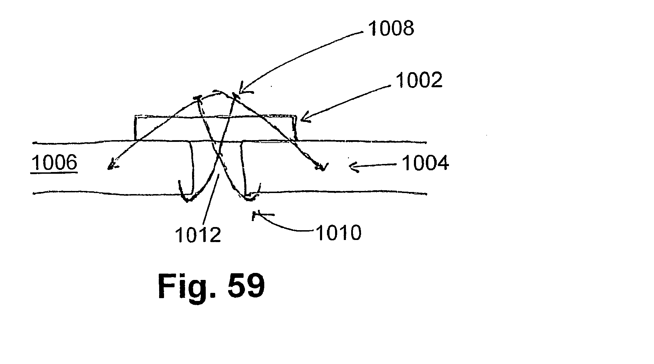

FIG 59 describes an embodiment where the patch element **1002** takes the form of a mesh seal. The securement is effected by a hook having a barb element **1004** that penetrates the inner surface of the annulus **1006,** while the inner connection of the hook (barb) **1004** is attached to the patch in such a fashion as to add tension between the outer surface of the annulus and the inner surface in proximity to the patch, thus drawing the annular tissues together. The patch/stent **1002** contains a spring ribbon element **1008** which can be formed from nitinol or other spring material. Hooks **1010** are then deployed to "grab" the annulus, either through penetration or through grasping into the aperture **1012** as shown.

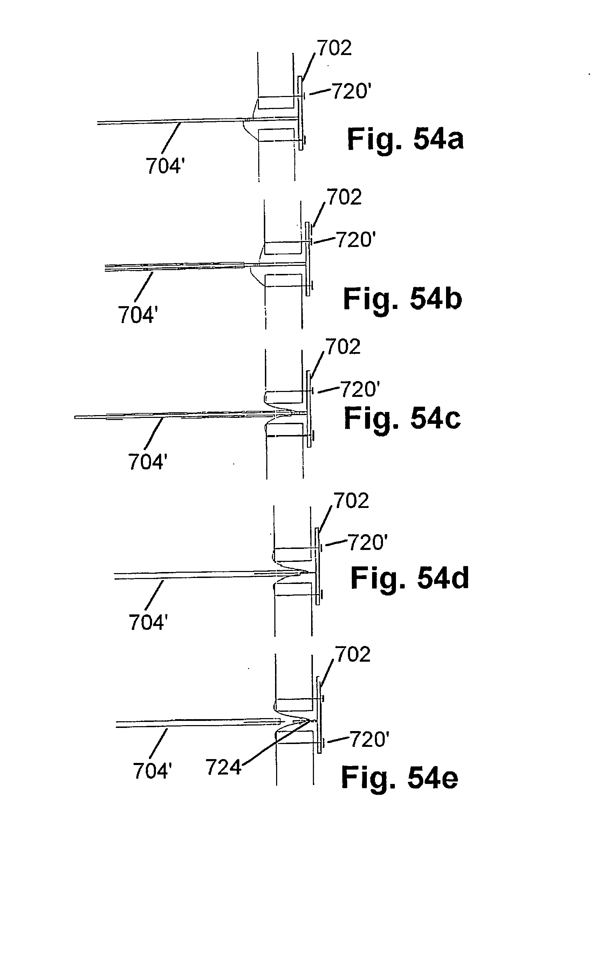

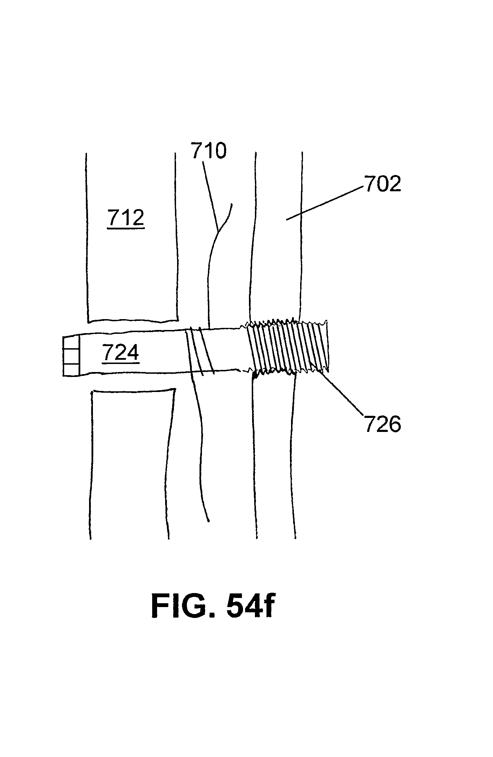

Figs. 54a-f shows another embodiment of a means to draw the suture lines together to cause tension between the inner and outer tissues of the annulus. Anchor bands, for example T-barbs **720'** are placed through the annulus and the patch, and they are secured to the patch **702.** "Slack" in the suture of the anchor band is "rotated" around a detachable portion of the delivery tool **704'** and a locking element, for example a screw configuration **724** as shown in the drawing, is used to lock the extra suture line in place affixed to threads **726** with the patch **702.** The delivery tool **704'** is then removed.

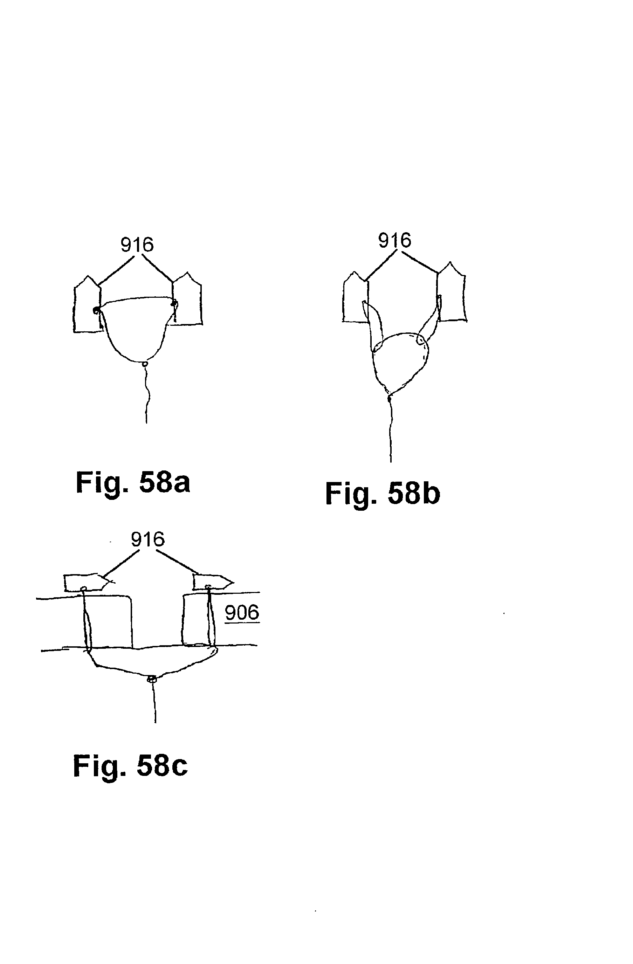

Fig. 58 shows alternative embodiments for tightening "anchoring barbs" with different configurations of sutures and cinch lines. For example in Fig. 58B each independent barb has a looped suture attached to it. Through each of these loops is passed a cinch line, which contains a knot. After placement of the barbs within the annulus, and possibly through the patch, the cinch line draws the loops of the barbs together. The advantage of this embodiment is that it allows for the independent placement of multiple barbs and the ability to draw all of them together.

Although cinch lines have been described as using a knot to "lock" the length of the suture, other mechanisms could also lock the length, as shown in Figure 57. The locking of the suture length is accomplished through a mechanical element located on the barb which engages with three dimensional elements attached to the suture line which mechanically press fit through the engagement element on the barb, thus locking the length of the suture line into place.

Although the embodiments of Fig. 57 and Fig. 58 depict the use of a single locking mechanism (e.g., knot on cinch line), it is conceivable that various designs could use more than one locking element to achieve the re-approximation and drawing together the tissue surrounding an aperture.

All patents referred to or cited herein are relevant in their entirety to the extent they are not inconsistent with the explicit teachings of this specification, including; U.S. Patent No. 5,108,438 (Stone), U.S. Patent No. 5,258,043 (Stone), U.S. Patent No. 4,904,260 (Ray et al.), U.S. Patent No. 5,964,807 (Gan et al.), U.S. Patent No. 5,849,331 (Ducheyne et al.), U.S. Patent No. 5,122,154 (Rhodes), U.S. Patent No. 5,204,106 (Schepers at al.), U.S. Patent No. 5,888,220 (Felt et al.) and U.S. Patent No. 5,376,120 (Sarver et al.).

Various materials know to those skilled in the art can be employed in practicing the present invention. By means of example only, the body portions of the stent could be made of NiTi alloy, plastics including polypropylene and polyethylene, stainless steel and other biocompatible metals, chromium cobalt alloy, or collagen. Webbing materials can include silicone, collagen, ePTFE, DACRON, polyester, polypropylene, polyethylene, and other biocompatible materials and can be woven or non-woven. Membranes might be fashioned of silicone, propylene, polyester, SURLYN, PEBAX, polyethylene, polyurethane or other biocompatible materials. Inflation fluids for membranes can include gases, liquids, foams, emulsions, and can be or contain bioactive materials and can also be for mechanical, biochemical and medicinal purposes. The stent body, webbing and/or membrane can be drug eluting or bioabsorbable, as known in the medical implant arts.

Other embodiments of the invention will be apparent to those skilled in the art from consideration of the specification and practice of the invention disclosed herein. It is intended that the specification and examples be considered as exemplary only, with the scope of the invention being indicated by the following claims.

## Claims

1. A device (120, 702) for occluding an aperture (44, 116, 208, 408, 1012) in the wall (610) of an annulus fibrosus (42, 206, 402, 612, 712, 906, 1006) and preventing the migration of nucleus pulposus (203) therethrough, the device (120, 702) being expandable, and the device (120, 702) having
a first collapsed configuration dimensioned to pass through said aperture (44, 116, 208, 408, 1012);
a second expanded configuration having at least one dimension at least as large as said aperture (44, 116, 208, 408, 1012) and having at least one dimension larger than a corresponding dimension in said first collapsed configuration; **characterized by**, the device further comprising:
at least one anchoring band (144, 709, 709', 709"), each anchoring band having two barbed elements (134, 136, 709") that are connected by means of a connecting element (144, 709'), such as a suture (709') or a band (144); and
a fixation feature (124) mounted on the device (120, 702) such that the device (120, 702) may be captured on a delivery tool (122, 704),
an attachment element (142, 145, 710, 714), for example, a tether, a suture, a cinch line, or a staple, for attaching the at least one anchoring band (144, 709, 709', 709") to the device (120, 702).

2. The device (120, 702) according to claim 1, wherein the fixation feature (124) is configured so that the attachment element (142, 145, 710, 714) may be utilized to attach the fixation feature (124) of the device (120, 702) to the at least one anchoring band (134, 136, 144, 709, 709', 709"), wherein the fixation feature (124) preferably is a ring (124).

3. The device (120, 702) according to one of the preceding claims, wherein the at least one anchoring band (134, 136, 144, 709, 709', 709") provides a fixation site to draw the device (120, 702) within proximity of the sub-annular wall when the at least one anchoring band is affixed to a location surrounding the aperture, such as the annulus (712), or vertebral bodies (800), or Sharpey's Fibers (802).

4. The device according to one of the preceding claims, wherein the fixation feature (124) is configured so that the device (120, 702) may be captured on a delivery tool (122, 704) through the use of the fixation feature (124) for the purpose of inserting the device (120, 702) through the aperture (44, 116, 208, 408, 1012) into the sub-annular space when the device (120, 702) is in the first collapsed configuration.

5. The device (120, 702) according to any preceding claim, **characterized in that** the device (120, 702)comprises an elongated centralized vertical extension (12) with an upper section (14) and a lower section (16), the lower section (16) of the centralized vertical extension comprising a left (20) and a right (22) lateral extension.

6. The device (120, 702) according to claim 5, wherein said lateral extension further comprises a compressible core affixed to said lower surface (30), forming a pyramid, cone, trapezoid, wedge, or bullet shape.

7. The device (120, 702) according to claim 5, wherein the device (120, 702) further comprises a flexible bladder (60, 80) affixed to said lower surface (30) of said left and right lateral extensions (20, 22).

8. The device (120, 702) according to any preceding claim, **characterized in that** the device (120, 702) comprises multiple components to help with expansion of the device (120, 702) and a covering to obtain biocompatibility and tissue ingrowth.

9. The device (120, 702) according to claim 8, **characterized in that** at least one of said multiple components comprises a frame (502).

10. The device (120, 702) according to any preceding claim, **characterized in that** the device (120, 702) comprises at least in part a material selected from the group consisting of human muscle fascia, an autograft, an allograft or a xenograft.

## Patentansprüche

1. Eine Vorrichtung (120, 702) zum Verschließen einer Öffnung (44, 116, 208, 408, 1012) in der Wand (610) einer Anulus fibrosus (42, 206, 402, 612, 712, 906, 1006) und Verhindern der Migration von Nucleus pulposus (203) durch die Öffnung (44, 116, 208, 408, 1012), wobei die Vorrichtung (120, 702) ausdehnbar ist, und die Vorrichtung (120, 702) umfasst
eine erste kollabierte Konfiguration, welche dimensioniert ist, um die Öffnung (44, 116, 208, 408, 1012) zu passieren;
eine zweite ausgedehnte Konfiguration, welche zumindest eine Dimension aufweist, welche mindestens so groß wie die Öffnung (44, 116, 208, 408, 1012) ist und zumindest eine Dimension aufweist, welche größer als eine entsprechende Dimension in der ersten kollabierten Konfiguration ist; **dadurch gekennzeichnet, dass** die Vorrichtung weiter umfasst:
mindestens ein Ankerband (144, 709, 709', 709"), wobei jedes Ankerband zwei Elemente mit Widerhaken (134, 136, 709") aufweist, welche mittels eines Verbindungselements (144, 709'), wie zum Beispiel einem Faden (709') oder einem Band (144) verbunden sind; und
ein Fixierungsmerkmal (124), welches auf der Vorrichtung (120, 702) aufmontiert ist, so dass die Vorrichtung (120, 702) auf einem Zuführwerkzeug (122, 704) aufgenommen werden kann,
ein Befestigungselement (142, 145, 710, 714), zum Beispiel ein Haltegurt, ein Faden, eine Cinchleine, oder eine Klammer, zum Befestigen des mindestens einen Ankerbands (144, 709, 709', 709") an der Vorrichtung (120, 702).

2. Die Vorrichtung (120, 702) gemäß Anspruch 1, wobei das Fixierungsmerkmal (124) dazu konfiguriert ist, dass das Befestigungselement (142, 145, 710, 714) dazu verwendet werden kann, das Fixierungsmerkmal (124) der Vorrichtung (120, 702) an dem mindestens einen Ankerband (134, 136, 144, 709, 709', 709") zu befestigen, wobei das Fixierungsmerkmal (124) bevorzugt ein Ring (124) ist.

3. Die Vorrichtung (120, 702) gemäß einem der vorhergehenden Ansprüche, wobei das mindestens eine Ankerband (134, 136, 144, 709, 709', 709") eine Fixierungsstelle bereitstellt, um die Vorrichtung (120, 702) in die Nähe der nahezu ringförmigen Wand zu ziehen, wenn das mindestens eine Ankerband an einer die Öffnung umgebende Stelle angebracht ist, wie zum Beispiel den Anuli (712), oder den vertebral Körpern (800), oder den Sharpey' Fasern (802).

4. Die Vorrichtung gemäß einem der vorhergehenden Ansprüche, wobei das Fixierungsmerkmal (124) derart konfiguriert ist, dass die Vorrichtung (120, 702) durch Verwendung des Fixierungsmerkmals (124) auf einem Zuführwerkzeug (122, 704) aufgenommen werden kann zu dem Zweck die Vorrichtung (120, 702) durch die Öffnung (44, 116, 208, 408, 1012) in den nahezu ringförmigen Raum einzusetzen, wenn sich die Vorrichtung (120, 702) in der ersten kollabierten Konfiguration befindet.

5. Die Vorrichtung (120, 702) gemäß einem der vorhergehenden Ansprüche, **dadurch gekennzeichnet, dass** die Vorrichtung (120, 702) eine längliche, zentrierte, vertikale Ausdehnung (12) mit einem oberen Bereich (14) und einem unteren Bereich (16) umfasst, wobei der untere Bereich (16) der länglichen, zentrierten, vertikalen Ausdehnung eine linke (20) und eine rechte (22) seitliche Ausdehnung umfasst.

6. Die Vorrichtung (120, 702) gemäß Anspruch 5, wobei die seitliche Ausdehnung weiter einen komprimierbaren Kern umfasst, welcher an der unteren Oberfläche (30) angebracht ist und eine Pyramiden-, Kegel-, Trapezoid-, Keil- oder Kugelform bildet.

7. Die Vorrichtung (120, 702) gemäß Anspruch 5, wobei die Vorrichtung (120, 702) weiter einen flexiblen Balg (60, 80) umfasst, welcher an der unteren Oberfläche (30) der linken und rechten seitlichen Ausdehnungen (20, 22) angebracht ist.

8. Die Vorrichtung (120, 702) gemäß einem der vorhergehenden Ansprüche, **dadurch gekennzeichnet, dass** die Vorrichtung (120, 702) eine Vielzahl an Komponenten zur Hilfe bei der Ausdehnung der Vorrichtung (120, 702) und eine Abdeckung, um eine Biokompatibilität und Gewebeeinwuchs zu erhalten, umfasst.

9. Die Vorrichtung (120, 702) gemäß Anspruch 8, **dadurch gekennzeichnet, dass** zumindest eine der Vielzahl an Komponenten einen Rahmen (502) umfasst.

10. Die Vorrichtung (120, 702) gemäß einem der Vorhergehenden Ansprüche, **dadurch gekennzeichnet, dass** die Vorrichtung (120, 702) zumindest teilweise ein Material umfasst, welches aus der Gruppe ausgewählt ist, die aus menschlicher Muskelfaszie, einem Autotransplantat, einem Allotransplantat oder einem Xenotransplantat besteht.

## Revendications

1. Dispositif (120, 702) d'occlusion d'une ouverture (44, 116, 208, 408, 1012) dans la paroi (610) d'un anneau fibreux (42, 206, 402, 612, 712, 906, 1006) et de prévention de la migration du noyau pulpeux (203) à travers celle-ci, le dispositif (120, 702) étant expansible, et le dispositif (120, 702) ayant
une première configuration repliée dimensionnée pour passer à travers ladite ouverture (44, 116, 208, 408, 1012) ;
une seconde configuration déployée ayant au moins une dimension au moins aussi grande que ladite ouverture (44, 116, 208, 408, 1012) et ayant au moins une dimension plus grande qu'une dimension correspondante dans ladite première configuration repliée ; **caractérisé en ce que** le dispositif comprend en outre :
au moins une bande d'ancrage (144, 709, 709', 709"), chaque bande d'ancrage ayant deux éléments barbelés (134, 136, 709") qui sont raccordés au moyen d'un élément de raccordement (144, 709'), tel qu'une suture (709') ou une bande (144) ; et
un support de fixation (124) monté sur le dispositif (120, 702) de telle sorte que le dispositif (120, 702) peut être capturé sur un outil de mise en place (122, 704),
un élément d'attache (142, 145, 710, 714), par exemple, une longe, une suture, une ligne de serrage, ou une agrafe, pour attacher la ou les bandes d'ancrage (144, 709, 709', 709") au dispositif (120, 702).

2. Dispositif (120, 702) selon la revendication 1, dans lequel le support de fixation (124) est configuré pour que l'élément d'attache (142, 145, 710, 714) puisse être utilisé pour attacher le support de fixation (124) du dispositif (120, 702) à la ou aux bandes d'ancrage (134, 136, 144, 709, 709', 709"), dans lequel le support de fixation (124) est de préférence un anneau (124).

3. Dispositif (120, 702) selon l'une quelconque des revendications précédentes, dans lequel la ou les bandes d'ancrage (134, 136, 144, 709, 709', 709") fournissent un site de fixation pour rapprocher le dispositif (120, 702) de la paroi sous-annulaire lorsque la ou les bandes d'ancrage sont fixées à un emplacement entourant l'ouverture, par exemple l'anneau (712), ou les corps vertébraux (800) ou les fibres de Sharpey (802).

4. Dispositif selon l'une quelconque des revendications précédentes, dans lequel le support de fixation (124) est conçu de telle manière que le dispositif (120, 702) peut être capturé sur un outil de mise en place (122, 704) par l'utilisation du support de fixation (124) afin d'insérer le dispositif (120, 702) à travers l'ouverture (44, 116, 208, 408, 1012) dans l'espace sous-annulaire lorsque le dispositif (120, 702) se trouve dans la première configuration repliée.

5. Dispositif (120, 702) selon l'une quelconque des revendications précédentes, **caractérisé en ce que** le dispositif (120, 702) comprend une extension verticale centralisée allongée (12) avec une section supérieure (14) et une section inférieure (16), la section inférieure (16) de l'extension verticale centralisée comprenant une extension latérale gauche (20) et une extension latérale droite (22).

6. Dispositif (120, 702) selon la revendication 5, dans lequel ladite extension latérale comprend en outre un coeur compressible fixé à ladite surface inférieure (30), formant une forme pyramidale, conique, trapézoïdale, triangulaire ou d'ogive.

7. Dispositif (120, 702) selon la revendication 5, dans lequel le dispositif (120, 720) comprend en outre une vessie souple (60, 80) fixée à ladite surface inférieure (30) desdites extensions latérales gauche et droite (20, 22).

8. Dispositif (120, 702) selon l'une quelconque des revendications précédentes, **caractérisé en ce que** le dispositif (120, 702) comprend de multiples composants pour aider au déploiement du dispositif (120, 702) et un revêtement pour permettre la biocompatibilité et la croissance du tissu.

9. Dispositif (120, 702) selon la revendication 8, **caractérisé en ce qu'**au moins l'un desdits multiples composants comprend un châssis (502).

10. Dispositif (120, 702) selon l'une quelconque des revendications précédentes, **caractérisé en ce que** le dispositif (120, 702) comprend au moins en partie un matériau sélectionné dans le groupe constitué d'un fascia musculaire humain, d'une autogreffe, d'une allogreffe ou d'une xénogreffe .
